(19) Europäisches Patentamt
European Patent Office
Office européen des brevets

(11) Veröffentlichungsnummer: **0 342 568 B1**

(12) **EUROPÄISCHE PATENTSCHRIFT**

(45) Veröffentlichungstag der Patentschrift: **02.02.94**

(51) Int. Cl.[5]: **C07D 239/52**, C07D 239/42, C07D 239/47, A01N 47/36

(21) Anmeldenummer: **89108701.7**

(22) Anmeldetag: **16.05.89**

Die Akte enthält technische Angaben, die nach dem Eingang der Anmeldung eingereicht wurden und die nicht in dieser Patentschrift enthalten sind.

(54) **Heterocyclisch substituierte Phenoxysulfonylharnstoffe, Verfahren zu ihrer Herstellung und ihre Verwendung als Herbizide oder Pflanzenwachstumsregulatoren.**

(30) Priorität: **17.05.88 DE 3816703**

(43) Veröffentlichungstag der Anmeldung:
**23.11.89 Patentblatt 89/47**

(45) Bekanntmachung des Hinweises auf die Patenterteilung:
**02.02.94 Patentblatt 94/05**

(84) Benannte Vertragsstaaten:
**BE CH DE ES FR GB IT LI NL SE**

(56) Entgegenhaltungen:
**EP-A- 0 004 163**
**EP-A- 0 094 790**
**EP-A- 0 113 956**
**DE-A- 3 151 450**

**CHEMICAL ABSTRACTS, Band 108, 1988, Seite 254, Zusammenfassung Nr. 200229s, Columbus, Ohio, US**

**CHEMICAL ABSTRACTS, Band 108, 1988, Seite 249, Zusammenfassung Nr. 33622f, Columbus, Ohio, US**

(73) Patentinhaber: **HOECHST AKTIENGESELLSCHAFT**

**D-65926 Frankfurt(DE)**

(72) Erfinder: **Kehne, Heinz, Dr.**
**Berliner Strasse 10**
**D-6238 Hofheim am Taunus(DE)**
Erfinder: **Willms, Lothar, Dr.**
**Schulstrasse 3**
**D-5411 Hillscheid(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Eppstein/Taunus(DE)**
Erfinder: **Bürstell, Helmut, Dr.**
**Am Hohlacker 65**
**D-6000 Frankfurt am Main 50(DE)**

EP 0 342 568 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß heterocyclisch substituierte Phenoxysulfonylharnstoffe herbizide und pflanzenwachstumsregulierende Eigenschaften besitzen (EP-A 4163, EP-A 113,956, DE-A 3151-450).

Es wurde nun gefunden, daß heterocyclisch substituierte Sulfamidsäurephenylester, deren Phenylesterteil von speziellen Brenzcatechinmonoethern gebildet wird, als Herbizide und Pflanzenwachstumsregulatoren besonders geeignet sind.

Gegenstand der vorliegenden Erfindung sind daher Verbindungen der Formel (I) oder deren Salze

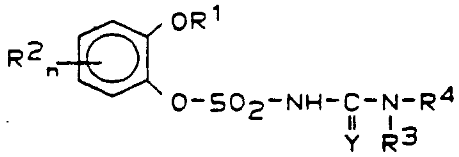

(I),

worin

- $R^1$ ($C_1$-$C_8$)Alkyl, ($C_2$-$C_8$)Alkenyl, ($C_2$-$C_8$)Alkinyl, wobei diese Reste jeweils ein- oder mehrfach durch Chlor oder Brom ein- oder zweifach durch ($C_1$-$C_6$)Alkoxy, ($C_2$-$C_6$)Alkenyloxy, ($C_2$-$C_6$)-Alkinyloxy, ($C_1$-$C_6$)Alkylthio, ($C_1$-$C_6$)Alkylsulfinyl, ($C_1$-$C_6$)Alkylsulfonyl, ($C_1$-$C_6$)Alkoxycarbonyl, $NO_2$, CN oder durch Phenyl substituiert sind; ferner ($C_2$-$C_8$)Alkenyl, ($C_2$-$C_8$)Alkinyl, ($C_3$-$C_8$)-Cycloalkyl, das ein- oder mehrfach durch Halogen oder ein- oder zweifach durch ($C_1$-$C_4$)-Alkoxy oder ($C_1$-$C_4$)Alkylthio substituiert sein kann; ($C_5$-$C_8$)Cycloalkenyl, Cyclopropylmethyl, Epoxypropyl; Furfuryl, Tetrahydrofurfuryl, Phenoxy($C_1$-$C_4$)alkyl, wobei die drei letztgenannten Substituenten durch Halogen, ($C_1$-$C_4$)Alkyl, ($C_1$-$C_4$)Alkoxy substituiert sein können; oder Phenyl, das ein- oder mehrfach durch Halogen, ($C_1$-$C_4$)Alkyl, ($C_1$-$C_4$)Alkoxy, $NO_2$, $CF_3$, CN oder $OCHF_2$ substituiert sein kann;
  oder für den Fall, daß $R^2$ ($C_2$-$C_8$)Alkenyl, ($C_2$-$C_8$)Alkinyl, Phenyl, Phenoxy, die wie unten angegeben substituiert sein können, ($C_1$-$C_4$)Alkylsulfonyl oder ($C_1$-$C_4$)Alkylsulfinyl bedeutet und $n \neq 0$ ist, $R^1$ auch ($C_1$-$C_8$)Alkyl bedeutet,
- $R^2$ unabhängig voneinander ($C_1$-$C_8$)Alkyl, ($C_2$-$C_8$)-Alkenyl, ($C_2$-$C_8$)Alkinyl, Phenyl, Phenoxy, ($C_1$-$C_4$)Alkoxy, ($C_1$-$C_4$)-Alkylthio, ($C_1$-$C_4$)Alkoxycarbonyl, ($C_2$-$C_4$)Alkenyloxycarbonyl, ($C_2$-$C_4$)-Alkinyloxycarbonyl, wobei alle vorgenannten Reste ein- oder mehrfach durch Halogen oder ein- bis zweifach durch ($C_1$-$C_4$)Alkoxy oder ($C_1$-$C_4$)-Alkylthio substituiert sein können; ferner Halogen, $NO_2$, ($C_1$-$C_4$)-Alkylsulfonyl oder ($C_1$-$C_4$)-Alkylsulfinyl;
- n 0, 1, 2 oder 3;
- y O oder S;
- $R^3$ Wasserstoff, ($C_1$-$C_8$)Alkyl, ($C_2$-$C_8$)Alkenyl, ($C_3$-$C_8$)Alkinyl oder ($C_1$-$C_4$)Alkoxy;
- $R^4$ einen heterocyclischen Rest der Formeln

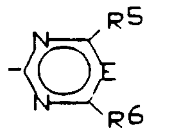 , 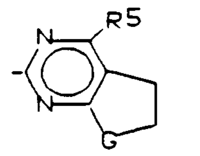 , 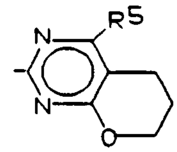 ,

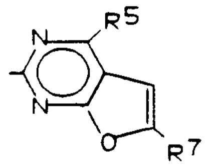 , 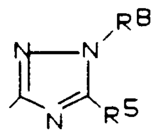 , 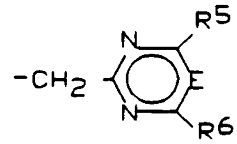 ,

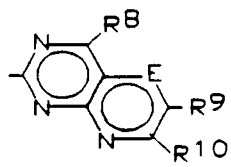 oder 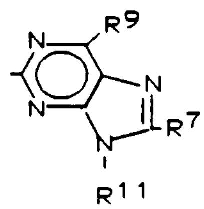

| | |
|---|---|
| E | CH oder N, |
| G | O oder $CH_2$, |
| $R^5$, $R^6$ | unabhängig voneinander Wasserstoff, Halogen, $(C_1\text{-}C_6)$-Alkyl, $(C_1\text{-}C_6)$Alkoxy oder $(C_1\text{-}C_6)$-Alkylthio, wobei die vorgenannten alkylhaltigen Reste ein- oder mehrfach durch Halogen oder ein- oder zweifach durch $(C_1\text{-}C_4)$Alkoxy oder $(C_1\text{-}C_4)$Alkylthio substituiert sein können, ferner einen Rest der Formel $-NR^{12}R^{13}$, $-OCHR^7\text{-}CO_2R^{12}$, $(C_3\text{-}C_6)$Cycloalkyl, $(C_3\text{-}C_5)$Alkenyl, $(C_2\text{-}C_4)$Alkinyl, $(C_3\text{-}C_5)$- Alkenyloxy oder $(C_3\text{-}C_5)$Alkinyloxy, |
| $R^7$ | Wasserstoff oder $(C_1\text{-}C_4)$Alkyl, |
| $R^8$ | $(C_1\text{-}C_4)$Alkyl, $-CHF_2$ oder $-CH_2CF_3$, |
| $R^9$, $R^{10}$ | unabhängig voneinander Wasserstoff, $(C_1\text{-}C_4)$Alkyl, $(C_1\text{-}C_5)$Alkoxy oder Halogen, |
| $R^{11}$ | Wasserstoff, $(C_1\text{-}C_4)$Alkyl, $-CHF_2$ oder $CH_2CF_3$ und |
| $R^{12}$, $R^{13}$ | unabhängig voneinander Wasserstoff, $(C_1\text{-}C_4)$Alkyl, $(C_2\text{-}C_4)$Alkenyl oder $(C_3\text{-}C_4)$Alkinyl |

bedeuten.

Die Verbindungen der Formel (I) können Salze bilden, bei denen der Wasserstoff der $-SO_2$-NH-Gruppe durch ein für die Landwirtschaft geeignetes Kation ersetzt wird. Diese Salze sind im allgemeinen Metall-, insbesondere Alkali-, Erdalkali-, gegebenenfalls alkylierte Ammonium- oder organische Aminsalze.

Bevorzugte Verbindungen der Formel (I) und deren Salze sind solche, bei denen $R^1$ $(C_1\text{-}C_4)$Alkyl, $(C_2\text{-}C_5)$Alkenyl oder $(C_2\text{-}C_4)$Alkinyl, wobei diese Reste wie oben beschrieben substituiert sind; ferner $(C_2\text{-}C_5)$-Alkenyl, $(C_2\text{-}C_4)$Alkinyl oder für den Fall, daß n = 1 und $R^2$ = $(C_2\text{-}C_8)$Alkenyl oder $(C_2\text{-}C_8)$Alkinyl ist, $(C_1\text{-}C_4)$Alkyl; $R^2$ $(C_1\text{-}C_4)$-Alkyl, $(C_2\text{-}C_5)$Alkenyl, $(C_2\text{-}C_4)$Alkinyl, $(C_1\text{-}C_4)$Alkoxycarbonyl, Phenyl, Phenoxy, $(C_1\text{-}C_4)$-Alkoxy, $(C_1\text{-}C_4)$Alkylthio, wobei diese Reste wie oben beschrieben substituiert sein können; oder Halogen; n 0, 1 oder 2, Y = O, $R^3$ Wasserstoff, $(C_1\text{-}C_4)$Alkyl oder $(C_3\text{-}C_4)$Alkenyl, $R^4$ einen heterocyclischen Rest der Formel

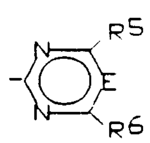 ,

E = CH oder N ,
$R^5$ und $R^6$ unabhängig voneinander Halogen, $(C_1\text{-}C_4)$Alkyl, $(C_1\text{-}C_4)$Alkoxy, $(C_1\text{-}C_4)$Alkylthio, die wie oben beschrieben substituiert sein können, bedeuten.

Halogen bedeutet vorzugsweise Fluor, Chlor oder Brom.

Besonders bevorzugte Verbindungen der Formel (I) und deren Salze sind solche, worin $R^1$ $(C_1\text{-}C_4)$Alkyl, $(C_2\text{-}C_5)$Alkenyl, $(C_2\text{-}C_4)$Alkinyl, wobei diese Reste wie oben beschrieben substituiert sind, ferner $(C_2\text{-}C_5)$-Alkenyl oder $(C_2\text{-}C_4)$Alkinyl; $R^2$ $(C_1\text{-}C_4)$Alkyl, $(C_2\text{-}C_5)$Alkenyl, $(C_1\text{-}C_4)$-Alkoxycarbonyl, $(C_1\text{-}C_4)$Alkoxy, $(C_1\text{-}C_4)$Alkylthio, die wie oben beschrieben substituiert sein können; Fluor oder Chlor, n = 0 oder 1, $R^3$ Wasserstoff oder Methyl, $R^4$ einen heterocyclischen Rest der Formel

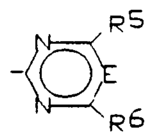

E = CH oder N
und $R^5$ und $R^6$ unabhängig voneinander Chlor, Brom, $(C_1\text{-}C_4)$Alkyl, $(C_1\text{-}C_4)$Alkoxy, $OCHF_2$, $OCH_2CF_3$ oder $CF_3$ bedeuten.

Weiterer Gegenstand der vorliegenden Erfindung sind Verfahren zur Herstellung der Verbindungen der allgemeinen Formel (I) oder deren Salze dadurch gekennzeichnet, daß man

(a) eine Verbindung der Formel (II)

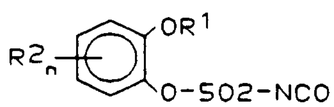

(II)

mit einer Verbindung der Formel (III)

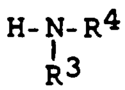

(III)

umsetzt, oder

(b) eine Verbindung der Formel (IV)

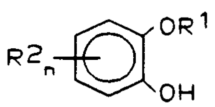

(IV)

mit einem Chlorsulfonylharnstoff der Formel (V)

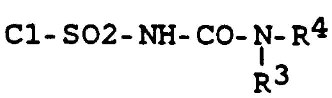

(V)

umsetzt, oder

(c) eine Verbindung der Formel (VI)

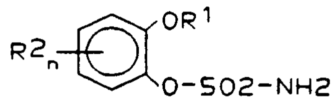

(VI)

mit einem Carbamat der Formel (VII)

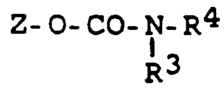

(VII)

worin Z Phenyl oder ($C_1$-$C_6$)Alkyl bedeutet, umsetzt, und die erhaltenen Verbindungen der Formel I gegebenenfalls in ihre Salze überführt.

Die Umsetzung der Verbindungen der Formeln (II) und (III) erfolgt vorzugsweise in inerten aprotischen Lösungsmitteln, wie z.B. Acetonitril, Dichlormethan, Toluol, Tetrahydrofuran oder Dioxan bei Temperaturen zwischen 0 °C und der Siedetemperatur des Lösungsmittels.

Die Phenoxysulfonylisocyanate der Formeln (II) lassen sich nach im Prinzip bekannten Verfahren aus den entsprechenden Brenzcatechinmonoethern der Formel (IV) und Chlorsulfonylisocyanat in einfacher Weise herstellen (vgl. G. Lohaus, Chem. Ber. 105, 2791 (1972)).

Die Ausgangsstoffe der Formel (III) sind bekannt oder lassen sich nach im Prinzip bekannten Verfahren herstellen, z.B. durch Cyclisierung entsprechender Guanidinderivate mit entsprechend substituierten 1,3-Diketonen, vergleiche z. B. "The Chemistry of Heterocyclic Compounds", Vol. XVI (1962) and Supplement I (1970), oder durch Derivatisierung von Cyanurchlorid, vgl. z. B. "The Chemistry of Heterocyclic Compounds", L. Rapaport: "s-Triazines and Derivatives" (1959).

Die Umsetzung der Verbindungen (IV) mit den Chlorsulfonylharnstoffen (V) führt man vorzugsweise in inerten Lösungsmitteln wie z. B. Dichlormethan bei Temperaturen zwischen -10 °C und 80 °C in Gegenwart einer Base als HCl-bindendes Agens durch. Als Basen können Alkali- oder Erdalkalicarbonate bzw. -bicarbonate wie z. B. $K_2CO_3$, $NaHCO_3$, $Na_2CO_3$ oder tertiäre Amine wie z. B. Pyridin oder Triethylamin eingesetzt werden.

Die Brenzcatechinmonoether (IV) sind literaturbekannt oder können nach literaturbekannten Verfahren hergestellt werden. Die Chlorsulfonylharnstoffe (V) sind aus den Aminen der Formel (III) und Chlorsulfonylisocyanat zugänglich (EP-A 141 199).

Die Umsetzung der Verbindungen (VI) mit den heterocyclischen Carbamaten der Formel (VII) wird vorzugsweise in Gegenwart von tertiären organischen Basen wie z. B. 1,8-Diazabicyclo[5.4.0]undec-7-en (DBU) in inerten Lösungsmitteln wie Acetonitril oder Dioxan bei Temperaturen zwischen 20 °C und der Siedetemperatur des Lösungsmittels durchgeführt (analog EP-A 44 807).

Die hierzu erforderlichen Carbamate (VII) sind literaturbekannt oder werden nach bekannten Verfahren hergestellt (EP-A 70 804). Die Sulfamate (VI) werden nach bekannten Verfahren aus den zugrundeliegenden Brenzcatechinmonoethern hergestellt (vgl. z. B. Synthesis 1978, 357; Z. Chem. 15, 270 (1975); Chem. Ber. 105, 2791 (1972)).

Die Salze der Verbindungen der Formel I werden vorzugsweise in inerten Lösungmitteln wie z.B. Wasser, Methanol oder Aceton bei Temperaturen von 0 - 100 °C hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöckchen oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon, Sida auf der annuellen Seite sowie Convolvulus, Cirsium, Rumex, Artemisia bei den Perennierenden.

Unter den spezifischen Kulturbedingungen im Reis vorkommende Unkräuter wie z.B. Sagittaria, Alisma, Eleocharis, Scirpus, Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verwendungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, Emulsionen, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder duch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw.

verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

## Formulierungsbeispiele

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz-und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (®Triton X 207), 3 Gewichtsteilen Isotridecanolpolyglykolether (8 EO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 EO) als Emulgator.

## CHEMISCHE BEISPIELE

### BEISPIEL 1

2-(2-Chlorethoxy)phenoxysulfonylisocyanat

Zu einer Lösung von 3,45 g (0,02 mol) 2-(2-Chlorethoxy)phenol in 20 ml Xylol tropft man bei 25 °C 3,4 g (0,024 mol) Chlorsulfonylisocyanat. Nach Beendigung des Zutropfens steigert man die Temperatur langsam auf 140 °C und erhitzt 2 h unter Rückfluß. Man kühlt ab und entfernt das Lösungsmittel sowie überschüssiges Chlorsulfonylisocyanat am Rotationsverdampfer. Das zurückbleibende gelbe Öl (5,6 g ≙ 100 % d.Th.) wird ohne weitere Reinigung eingesetzt.

### BEISPIEL 2

1-[2-(2-Chlorethoxy)phenoxysulfonyl]-3-(4,6-dimethoxypyrimidin-2-yl)harnstoff

Zu 3,1 g (0,02 mol) 2-Amino-4,6-dimethoxypyrimidin in 30 ml Dichlormethan tropft man bei 0 °C eine Lösung aus 5,6 g (0,02 mol) des Produkts aus Beispiel 1 in 20 ml Dichlormethan. Man läßt das Reaktionsgemisch auf Raumtemperatur erwärmen und rührt 24 h nach. Die Reaktionslösung wird mit 100 ml Dichlormethan verdünnt, mit 50 ml 1 N Salzsäure und 50 ml Wasser gewaschen. Man trocknet die organische Phase mit Natriumsulfat und entfernt das Lösungsmittel am Rotationsverdampfer. Das hinterbleibende viskose Öl wird durch Verreiben mit Diethylether kristallisiert. Man erhält 7,7 g (89 % d. Th.) 1-[2-(2-Chlorethoxy)phenoxysulfonyl]-3-(4,6-dimethoxypyrimidin-2-yl)harnstoff vom Schmp. 132-134 °C.

**BEISPIEL 3**

3-(4,6-Dimethoxypyrimidin-2-yl)-1-(2-propargyloxyphenoxysulfonyl)harnstoff

3,1 g (0,02 mol) 2-Amino-4,6-dimethoxypyrimidin werden in 30 ml Dichlormethan gelöst und bei 0 °C mit 5,1 g (0,02 mol) 2-Propargyloxyphenoxysulfonylisocyanat-gelöst in 20 ml Dichlormethan-versetzt. Nach 24-stündigem Rühren bei Raumtemperatur wird das Reaktionsgemisch mit 50 ml Dichlormethan verdünnt, mit 50 ml 1N Salzsäure und 50 ml Wasser gewaschen, getrocknet und eingedampft. Der ölige Rückstand kristallisiert beim Verreiben mit Diethylether. Man erhält 7,4 g (91 % d. Th.) 3-(4,6-Dimethoxypyrimidin-2-yl)-1-(2-propargyloxyphenoxysulfonyl)harnstoff vom Schmp. 125-126 °C.

**BEISPIEL 4**

3-(4,6-Dimethoxypyrimidin-2-yl)-1-[2-(2-methoxyethoxy)phenoxysulfonyl]harnstoff

1,74 g (0,0063 mol) Phenyl-N-(4,6-dimethoxypyrimidin-2-yl)carbamat werden in 80 ml Acetonitril gelöst und bei Raumtemperatur mit 1,48 g (0,006 mol) 2-(2-Methoxyethoxy)phenylsulfamat versetzt. Nach Zugabe von 1,0 g (0,0066 mol) 1,8-Diazabicyclo[5.4.0]undec-7-en (DBU) wird das Reaktionsgemisch 18 h bei Raumtemperatur gerührt, eingeengt, mit H20 verdünnt und mit 2N Salzsäure auf pH 3-4 angesäuert. Nach Absaugen und Trocknen erhält man 2,4 g (93 % d. Th.) 3-(4,6-Dimethoxypyrimidin-2-yl)-1-[2-(2-methoxyethoxy)phenoxysulfonyl]harnstoff vom Schmp. 106-108 °C.

Die Verbindungen der nachfolgenden Tabellen werden auf analoge Weise wie in Beispielen 1-4 beschrieben, hergestellt.

**Tabelle 1**

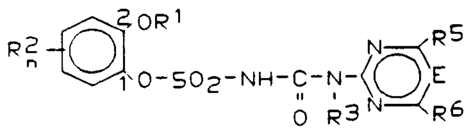

| Bsp.Nr. | $R^1$ | $R^3$ | $R^5$ | $R^6$ | E | n | Fp. [°C] |
|---|---|---|---|---|---|---|---|
| 5 | $CH_2CH_2Cl$ | H | $CH_3$ | $CH_3$ | CH | 0 | |
| 6 | " | H | $OCH_3$ | $CH_3$ | CH | 0 | |
| 7 | " | H | $CH_3$ | $CH_3$ | N | 0 | |
| 8 | " | H | $OCH_3$ | $CH_3$ | N | 0 | |
| 9 | " | H | $OCH_3$ | $OCH_3$ | N | 0 | |
| 10 | " | H | $OCH_3$ | Cl | CH | 0 | |
| 11 | " | H | $OCF_2H$ | $CH_3$ | CH | 0 | |
| 12 | " | H | $OCF_2H$ | $OCF_2H$ | CH | 0 | |
| 13 | " | H | $OCH_3$ | Br | CH | 0 | |
| 14 | " | H | $CH_3$ | Cl | CH | 0 | |
| 15 | " | H | $OCH_3$ | H | CH | 0 | |
| 16 | " | H | $OCH_3$ | $NHCH_3$ | CH | 0 | |
| 17 | " | H | $OCH_3$ | $NHCH_3$ | N | 0 | |
| 18 | " | H | $CH_3$ | $NHCH_3$ | CH | 0 | |
| 19 | " | H | $CH_3$ | $NHCH_3$ | N | 0 | |
| 20 | " | H | $OCH_3$ | $SCH_3$ | CH | 0 | |
| 21 | " | H | $OCH_3$ | $OC_2H_5$ | CH | 0 | |
| 22 | " | H | $OCH_3$ | $OC_3H_7$ | CH | 0 | |
| 23 | " | H | $OCH_3$ | $OC_2H_5$ | N | 0 | |
| 24 | " | H | Cl | $OC_2H_5$ | CH | 0 | |
| 25 | " | H | $OC_2H_5$ | $OC_2H_5$ | CH | 0 | |
| 26 | " | H | $C_2H_5$ | $OCH_3$ | CH | 0 | |
| 27 | " | H | $CF_3$ | $OCH_3$ | CH | 0 | |
| 28 | " | H | $OCH_2CF_3$ | $CH_3$ | CH | 0 | |
| 29 | " | H | $OCH_2CF_3$ | $OCH_3$ | CH | 0 | |
| 30 | " | H | $OCH_2CF_3$ | $OCH_2CF_3$ | CH | 0 | |
| 31 | " | H | $OCH_2CF_3$ | $NHCH_3$ | CH | 0 | |

Fortsetzung Tabelle 1

| Bsp.Nr. | $R^1$ | $R^3$ | $R^5$ | $R^6$ | E | n | Fp. [°C] |
|---|---|---|---|---|---|---|---|
| 32 | " | H | $OCH_2CF_3$ | $OCH_3$ | N | 0 | |
| 33 | " | H | $OCH_2CF_3$ | $NHCH_3$ | N | 0 | |
| 34 | " | H | $OCH_3$ | $NHC_2H_5$ | CH | 0 | |
| 35 | " | H | $OCH_2CF_3$ | $NHC_2H_5$ | CH | 0 | |
| 36 | " | H | $OCH_3$ | $N(CH_3)_2$ | CH | 0 | |
| 37 | " | H | $OCH_3$ | $CH(OCH_3)_2$ | CH | 0 | |
| 38 | $CH_2CH_2OCH_3$ | H | $CH_3$ | $CH_3$ | CH | 0 | 103-105 |
| 39 | " | H | $OCH_3$ | $CH_3$ | CH | 0 | |
| 40 | " | H | $CH_3$ | $CH_3$ | N | 0 | |
| 41 | " | H | $OCH_3$ | $CH_3$ | N | 0 | |
| 42 | " | H | $OCH_3$ | $OCH_3$ | N | 0 | |
| 43 | " | H | $OCH_3$ | Cl | CH | 0 | Harz |
| 44 | " | H | $OCF_2H$ | $CH_3$ | CH | 0 | |
| 45 | " | H | $OCF_2H$ | $OCF_2H$ | CH | 0 | |
| 46 | " | H | $OCH_3$ | Br | CH | 0 | |
| 47 | " | H | $CH_3$ | Cl | CH | 0 | |
| 48 | " | H | $OCH_3$ | H | CH | 0 | |
| 49 | " | H | $OCH_3$ | $NHCH_3$ | CH | 0 | |
| 50 | " | H | $OCH_3$ | $NHCH_3$ | N | 0 | |
| 51 | " | H | CH3 | $NHCH_3$ | CH | 0 | |
| 52 | " | H | $CH_3$ | $NHCH_3$ | N | 0 | |
| 53 | " | H | $OCH_3$ | $SCH_3$ | CH | 0 | |
| 54 | " | H | $OCH_3$ | $OC_2H_5$ | CH | 0 | |
| 55 | " | H | $OCH_3$ | $OC_3H_7$ | CH | 0 | |
| 56 | " | H | $OCH_3$ | $OC_2H_5$ | N | 0 | |
| 57 | " | H | Cl | $OC_2H_5$ | CH | 0 | |
| 58 | " | H | $OC_2H_5$ | $OC_2H_5$ | CH | 0 | |
| 59 | " | H | $C_2H_5$ | $OCH_3$ | CH | 0 | |
| 60 | " | H | $CF_3$ | $OCH_3$ | CH | 0 | |
| 61 | " | H | $OCH_2CF_3$ | $CH_3$ | CH | 0 | |
| 62 | " | H | $OCH_2CF_3$ | $OCH_3$ | CH | 0 | |
| 63 | " | H | $OCH_2CF_3$ | $OCH_2CF_3$ | CH | 0 | |

Fortsetzung Tabelle 1

| Bsp.Nr. | $R^1$ | $R^3$ | $R^5$ | $R^6$ | E | n | Fp. [°C] |
|---|---|---|---|---|---|---|---|
| 64 | $CH_2CH_2OCH_3$ | H | $OCH_2CF_3$ | $NHCH_3$ | CH | 0 | |
| 65 | " | H | $OCH_2CF_3$ | $OCH_3$ | N | 0 | |
| 66 | " | H | $OCH_2CF_3$ | $NHCH_3$ | N | 0 | |
| 67 | " | H | $OCH_3$ | $OCH_3$ | CH | 0 | |
| 68 | " | H | $OCH_2CF_3$ | $NHC_2H_5$ | CH | 0 | |
| 69 | " | H | $OCH_3$ | $N(CH_3)_2$ | CH | 0 | |
| 70 | " | H | $OCH_3$ | $CH(OCH_3)_2$ | CH | 0 | |
| 71 | $CH_2CH=CH_2$ | H | $CH_3$ | $CH_3$ | CH | 0 | 128-133 |
| 72 | " | H | $OCH_3$ | $CH_3$ | CH | 0 | 89-90 |
| 73 | " | H | $CH_3$ | $CH_3$ | N | 0 | |
| 74 | " | H | $OCH_3$ | $CH_3$ | N | 0 | 112-115 |
| 75 | " | H | $OCH_3$ | $OCH_3$ | N | 0 | |
| 76 | " | H | $OCH_3$ | Cl | CH | 0 | |
| 77 | " | H | $OCF_2H$ | $CH_3$ | CH | 0 | |
| 78 | " | H | $OCF_2H$ | $OCF_2H$ | CH | 0 | |
| 79 | " | H | $OCH_3$ | Br | CH | 0 | |
| 80 | " | H | $CH_3$ | Cl | CH | 0 | |
| 81 | " | H | $OCH_3$ | H | CH | 0 | |
| 82 | " | H | $OCH_3$ | $NHCH_3$ | CH | 0 | |
| 83 | " | H | $OCH_3$ | $NHCH_3$ | N | 0 | |
| 84 | " | H | $OCH_3$ | $OCH_3$ | CH | 0 | 123 |
| 85 | " | H | $CH_3$ | $NHCH_3$ | N | 0 | |
| 86 | " | H | $OCH_3$ | $SCH_3$ | CH | 0 | |
| 87 | " | H | $OCH_3$ | $OC_2H_5$ | CH | 0 | |
| 88 | " | H | $OCH_3$ | $OC_3H_7$ | CH | 0 | |
| 89 | " | H | $OCH_3$ | $OC_2H_5$ | N | 0 | |
| 90 | " | H | Cl | $OC_2H_5$ | CH | 0 | |
| 91 | " | H | $OC_2H_5$ | $OC_2H_5$ | CH | 0 | |
| 92 | " | H | $C_2H_5$ | $OCH_3$ | CH | 0 | |
| 93 | " | H | $CF_3$ | $OCH_3$ | CH | 0 | |
| 94 | " | H | $OCH_2CF_3$ | $CH_3$ | CH | 0 | |
| 95 | " | H | $OCH_2CF_3$ | $OCH_3$ | CH | 0 | |

**Fortsetzung Tabelle 1**

| Bsp.Nr. | $R^1$ | $R^3$ | $R^5$ | $R^6$ | E | n | Fp. [°C] |
|---|---|---|---|---|---|---|---|
| 96 | $CH_2CH=CH_2$ | H | $OCH_2CF_3$ | $OCH_2CF_3$ | CH | 0 | |
| 97 | " | H | $OCH_2CF_3$ | $NHCH_3$ | CH | 0 | |
| 98 | " | H | $OCH_2CF_3$ | $OCH_3$ | N | 0 | |
| 99 | " | H | $OCH_2CF_3$ | $NHCH_3$ | N | 0 | |
| 100 | " | H | $OCH_3$ | $NHC_2H_5$ | CH | 0 | |
| 101 | " | H | $OCH_2CF_3$ | $NHC_2H_5$ | CH | 0 | |
| 102 | " | H | $OCH_3$ | $N(CH_3)_2$ | CH | 0 | |
| 103 | " | H | $OCH_3$ | $CH(OCH_3)_2$ | CH | 0 | |
| 104 | $CH_2C≡CH$ | H | $OCH_3$ | Cl | N | 0 | |
| 105 | " | H | $CH_3$ | $CH_3$ | CH | 0 | 128-133 (Zers.) |
| 106 | " | H | $OCH_3$ | $CH_3$ | CH | 0 | 143-146 |
| 107 | " | H | $CH_3$ | $CH_3$ | N | 0 | |
| 108 | " | H | $OCH_3$ | $CH_3$ | N | 0 | |
| 109 | " | H | $OCH_3$ | $OCH_3$ | N | 0 | |
| 110 | " | H | $OCH_3$ | Cl | CH | 0 | |
| 111 | " | H | $OCF_2H$ | $CH_3$ | CH | 0 | |
| 112 | " | H | $OCF_2H$ | $OCF_2H$ | CH | 0 | |
| 113 | " | H | $OCH_3$ | Br | CH | 0 | |
| 114 | " | H | $CH_3$ | Cl | CH | 0 | |
| 115 | " | H | $OCH_3$ | H | CH | 0 | |
| 116 | " | H | $OCH_3$ | $NHCH_3$ | CH | 0 | |
| 117 | " | H | $OCH_3$ | $NHCH_3$ | N | 0 | |
| 118 | " | H | $CH_3$ | $NHCH_3$ | CH | 0 | |
| 119 | " | H | $CH_3$ | $NHCH_3$ | N | 0 | |
| 120 | " | H | $OCH_3$ | $SCH_3$ | CH | 0 | |
| 121 | " | H | $OCH_3$ | $OC_2H_5$ | CH | 0 | |
| 122 | " | H | $OCH_3$ | $OC_3H_7$ | CH | 0 | |
| 123 | " | H | $OCH_3$ | $OC_2H_5$ | N | 0 | |
| 124 | " | H | Cl | $OC_2H_5$ | CH | 0 | |
| 125 | " | H | $OC_2H_5$ | $OC_2H_5$ | CH | 0 | |
| 126 | " | H | $C_2H_5$ | $OCH_3$ | CH | 0 | |

Fortsetzung Tabelle 1

| Bsp.Nr. | $R^1$ | $R^3$ | $R^5$ | $R^6$ | E | n | Fp. [°C] |
|---|---|---|---|---|---|---|---|
| 127 | $CH_2C≡CH$ | H | $CF_3$ | $OCH_3$ | CH | 0 | |
| 128 | " | H | $OCH_2CF_3$ | $CH_3$ | CH | 0 | |
| 129 | " | H | $OCH_2CF_3$ | $OCH_3$ | CH | 0 | |
| 130 | " | H | $OCH_2CF_3$ | $OCH_2CF_3$ | CH | 0 | |
| 131 | " | H | $OCH_2CF_3$ | $NHCH_3$ | CH | 0 | |
| 132 | " | H | $OCH_2CF_3$ | $OCH_3$ | N | 0 | |
| 133 | " | H | $OCH_2CF_3$ | $NHCH_3$ | N | 0 | |
| 134 | " | H | $OCH_3$ | $NHC_2H_5$ | CH | 0 | |
| 135 | " | H | $OCH_2CF_3$ | $NHC_2H_5$ | CH | 0 | |
| 136 | " | H | $OCH_3$ | $N(CH_3)_2$ | CH | 0 | |
| 137 | " | H | $OCH_3$ | $CH(OCH_3)_2$ | CH | 0 | |
| 138 | $CH_2CCl=CH_2$ | H | $OCH_3$ | $OCH_3$ | CH | 0 | |
| 139 | " | H | $OCH_3$ | $CH_3$ | CH | 0 | |
| 140 | " | H | $OCH_3$ | $CH_3$ | N | 0 | |
| 141 | " | H | $OCH_3$ | $OCH_3$ | N | 0 | |
| 142 | " | H | $OCH_3$ | Cl | CH | 0 | |
| 143 | " | H | $OCF_2H$ | $CH_3$ | CH | 0 | |
| 144 | " | H | $OCF_2H$ | $OCF_2H$ | CH | 0 | |
| 145 | " | H | $OCH_3$ | $NHCH_3$ | CH | 0 | |
| 146 | " | H | Cl | $OC_2H_5$ | CH | 0 | |
| 147 | " | H | $OCH_2CF_3$ | $OCH_3$ | CH | 0 | |
| 148 | " | H | $OCH_2CF_3$ | $OCH_3$ | N | 0 | |
| 149 | $CH_2CH=CHCl$ | H | $OCH_3$ | $OCH_3$ | CH | 0 | |
| 150 | " | H | $OCH_3$ | $CH_3$ | CH | 0 | |
| 151 | " | H | $OCH_3$ | $CH_3$ | N | 0 | |
| 152 | " | H | $OCH_3$ | $OCH_3$ | N | 0 | |
| 153 | " | H | $OCH_3$ | Cl | CH | 0 | |
| 154 | " | H | $OCF_2H$ | $CH_3$ | CH | 0 | |
| 155 | " | H | $OCF_2H$ | $OCF_2H$ | CH | 0 | |
| 156 | " | H | $OCH_3$ | $NHCH_3$ | CH | 0 | |
| 157 | " | H | Cl | $OC_2H_5$ | CH | 0 | |
| 158 | " | H | $OCH_2CF_3$ | $OCH_3$ | CH | 0 | |

**Fortsetzung Tabelle 1**

| Bsp.Nr. | $R^1$ | $R^3$ | $R^5$ | $R^6$ | E | n | Fp. [°C] |
|---|---|---|---|---|---|---|---|
| 159 | $CH_2CH=CHCl$ | H | $OCH_2CF_3$ | $OCH_3$ | N | 0 | |
| 160 | $CH_2C(CH_3)=CH_2$ | H | $OCH_3$ | $OCH_3$ | CH | 0 | |
| 161 | " | H | $OCH_3$ | $CH_3$ | CH | 0 | |
| 162 | " | H | $OCH_3$ | $CH_3$ | N | 0 | |
| 163 | " | H | $OCH_3$ | $OCH_3$ | N | 0 | |
| 164 | " | H | $OCH_3$ | Cl | CH | 0 | |
| 165 | " | H | $OCF_2H$ | $CH_3$ | CH | 0 | |
| 166 | " | H | $OCF_2H$ | $CF_2H$ | CH | 0 | |
| 167 | " | H | $OCH_3$ | $NHCH_3$ | CH | 0 | |
| 168 | " | H | Cl | $OC_2H_5$ | CH | 0 | |
| 169 | " | H | $OCH_2CF_3$ | $OCH_3$ | CH | 0 | |
| 170 | " | H | $OCH_2CF_3$ | $OCH_3$ | N | 0 | |
| 171 | $CH_2CH=CHCH_3$ | H | $OCH_3$ | $OCH_3$ | CH | 0 | |
| 172 | " | H | $OCH_3$ | $CH_3$ | CH | 0 | |
| 173 | " | H | $OCH_3$ | $CH_3$ | N | 0 | |
| 174 | " | H | $OCH_3$ | $OCH_3$ | N | 0 | |
| 175 | " | H | $OCH_3$ | Cl | CH | 0 | |
| 176 | " | H | $OCF_2H$ | $CH_3$ | CH | 0 | |
| 177 | " | H | $OCF_2H$ | $OCF_2H$ | CH | 0 | |
| 178 | " | H | $OCH_3$ | $NHCH_3$ | CH | 0 | |
| 179 | " | H | Cl | $OC_2H_5$ | CH | 0 | |
| 180 | " | H | $OCH_2CF_3$ | $OCH_3$ | CH | 0 | |
| 181 | " | H | $OCH_2CF_3$ | $OCH_3$ | N | 0 | |
| 182 | $CH_2C\equiv CCH_3$ | H | $OCH_3$ | $OCH_3$ | CH | 0 | |
| 183 | " | H | $OCH_3$ | $CH_3$ | CH | 0 | |
| 184 | " | H | $OCH_3$ | $CH_3$ | N | 0 | |
| 185 | " | H | $OCH_3$ | $OCH_3$ | N | 0 | |
| 186 | " | H | $OCH_3$ | Cl | CH | 0 | |
| 187 | " | H | $OCF_2H$ | $CH_3$ | CH | 0 | |
| 188 | " | H | $OCF_2H$ | $OCF_2H$ | CH | 0 | |
| 189 | " | H | $OCH_3$ | $NHCH_3$ | CH | 0 | |
| 190 | " | H | Cl | $OC_2H_5$ | CH | 0 | |
| 191 | " | H | $OCH_2CF_3$ | $OCH_3$ | CH | 0 | |

**Fortsetzung Tabelle 1**

| Bsp.Nr. | $R^1$ | $R^3$ | $R^5$ | $R^6$ | E | n | Fp. [°C] |
|---|---|---|---|---|---|---|---|
| 192 | $CH_2C\equiv CCH_3$ | H | $OCH_2CF_3$ | $OCH_3$ | N | 0 | |
| 193 | $CH_2CH_2Br$ | H | $OCH_3$ | $OCH_3$ | CH | 0 | |
| 194 | " | H | $OCH_3$ | $CH_3$ | CH | 0 | |
| 195 | " | H | $OCH_3$ | $CH_3$ | N | 0 | |
| 196 | " | H | $OCH_3$ | $OCH_3$ | N | 0 | |
| 197 | " | H | $OCH_3$ | Cl | CH | 0 | |
| 198 | " | H | $OCF_2H$ | $CH_3$ | CH | 0 | |
| 199 | " | H | $OCF_2H$ | $OCF_2H$ | CH | 0 | |
| 100 | " | H | $OCH_3$ | $NHCH_3$ | CH | 0 | |
| 101 | " | H | Cl | $OC_2H_5$ | CH | 0 | |
| 102 | " | H | $OCH_2CF_3$ | $OCH_3$ | CH | 0 | |
| 203 | " | H | $OCH_2CF_3$ | $OCH_3$ | N | 0 | |
| 204 | $CH_2CH(OCH_3)_2$ | H | $OCH_3$ | $OCH_3$ | CH | 0 | |
| 205 | " | H | $OCH_3$ | $CH_3$ | CH | 0 | |
| 206 | " | H | $OCH_3$ | $CH_3$ | N | 0 | |
| 207 | $C_6H_5$ | H | $OCH_3$ | $OCH_3$ | CH | 0 | 117-119 |
| 208 | " | H | $OCH_3$ | $CH_3$ | CH | 0 | 138-139 |
| 209 | " | H | $OCH_3$ | $CH_3$ | N | 0 | 120-122 |
| 210 | " | H | $CH_3$ | Cl | CH | 0 | 147-148 |
| 211 | " | H | $OCH_3$ | Cl | CH | 0 | |
| 212 | " | H | $CH_3$ | $CH_3$ | CH | 0 | 136-138 |
| 213 | $CH_2C_6H_5$ | H | $OCH_3$ | $OCH_3$ | CH | 0 | |
| 214 | " | H | $OCH_3$ | $CH_3$ | CH | 0 | |
| 215 | $CCH_3CH=CH_2$ | H | $OCH_3$ | $OCH_3$ | CH | 0 | |
| 216 | " | H | $OCH_3$ | $CH_3$ | CH | 0 | |
| 217 | " | H | $OCH_3$ | $CH_3$ | N | 0 | |

**Fortsetzung Tabelle 1**

| Bsp.Nr. | $R^1$ | $(R^2)_n$ | $R^3$ | $R^5$ | $R^6$ | E | Fp. |
|---|---|---|---|---|---|---|---|
| 218 | $CH_2CH=CH_2$ | 6-$CH_3$ | H | $OCH_3$ | $OCH_3$ | CH | |
| 219 | " | 6-$CH_3$ | H | $OCH_3$ | $CH_3$ | CH | |
| 220 | " | 6-$CH_3$ | H | $OCH_3$ | $CH_3$ | N | |
| 221 | $CH_2CH_2Cl$ | 6-$CH_3$ | H | $OCH_3$ | $OCH_3$ | CH | |
| 222 | " | 6-$CH_3$ | H | $OCH_3$ | $CH_3$ | CH | |
| 223 | " | 6-$CH_3$ | H | $OCH_3$ | $CH_3$ | N | |
| 224 | $CH_2C\equiv CH$ | 6-$CH_3$ | H | $OCH_3$ | $OCH_3$ | CH | |
| 225 | " | 6-$CH_3$ | H | $OCH_3$ | $CH_3$ | CH | |
| 226 | " | 6-$CH_3$ | H | $OCH_3$ | $CH_3$ | N | |
| 227 | $CH_2CH_2OCH_3$ | 6-$CH_3$ | H | $OCH_3$ | $OCH_3$ | CH | |
| 228 | " | 6-$CH_3$ | H | $OCH_3$ | $CH_3$ | CH | |
| 229 | " | 6-$CH_3$ | H | $OCH_3$ | $CH_3$ | N | |
| 230 | $CH_2C_6H_5$ | 6-$CH_3$ | H | $OCH_3$ | $OCH_3$ | CH | |
| 231 | " | 6-$CH_3$ | H | $OCH_3$ | $CH_3$ | CH | |
| 232 | " | 6-$CH_3$ | H | $OCH_3$ | $CH_3$ | N | |
| 233 | $CCH_3CH=CH_2$ | 6-$CH_3$ | H | $OCH_3$ | $OCH_3$ | CH | |
| 234 | " | 6-$CH_3$ | H | $OCH_3$ | $CH_3$ | CH | |
| 235 | " | 6-$CH_3$ | H | $OCH_3$ | $CH_3$ | N | |
| 236 | $CH_2CH=CH_2$ | 5-$CH_3$ | H | $OCH_3$ | $OCH_3$ | CH | |

| Bsp.Nr. | $R^1$ | $(R^2)_n$ | $R^3$ | $R^5$ | $R^6$ | E | Fp. |
|---|---|---|---|---|---|---|---|
| 237 | $CH_2CH=CH_2$ | 5-$CH_3$ | H | $OCH_3$ | $CH_3$ | CH | |
| 238 | $CH_2CH_2Cl$ | 5-$CH_3$ | H | $OCH_3$ | $CH_3$ | N | |
| 239 | " | 5-$CH_3$ | H | $OCH_3$ | $OCH_3$ | CH | |
| 240 | $CH_2CH_2OCH_3H$ | 5-$CH_3$ | H | $OCH_3$ | $CH_3$ | CH | |
| 241 | " | 5-$CH_3$ | H | $OCH_3$ | $CH_3$ | N | |
| 242 | $CH_2C\equiv CH$ | 5-$CH_3$ | H | $OCH_3$ | $OCH_3$ | CH | |
| 243 | " | 5-$CH_3$ | H | $OCH_3$ | $CH_3$ | CH | |
| 244 | $CH_2CH_2Br$ | 4-$CH_3$ | H | $OCH_3$ | $CH_3$ | N | |
| 245 | " | 4-$CH_3$ | H | $OCH_3$ | $OCH_3$ | CH | |
| 246 | $CH_2C\equiv CCH_3$ | 3-$CH_3$ | H | $OCH_3$ | $CH_3$ | CH | |
| 247 | " | 3-$CH_3$ | H | $OCH_3$ | $CH_3$ | N | |
| 248 | $CH_2CH=CH_2$ | 6-Cl | H | $OCH_3$ | $OCH_3$ | CH | |
| 249 | " | 6-Cl | H | $OCH_3$ | $CH_3$ | CH | |
| 250 | " | 6-Cl | H | $OCH_3$ | $CH_3$ | N | |
| 251 | $CH_2CH_2Cl$ | 6-Cl | H | $OCH_3$ | $OCH_3$ | CH | |
| 252 | " | 6-Cl | H | $OCH_3$ | $CH_3$ | CH | |
| 253 | " | 6-Cl | H | $OCH_3$ | $CH_3$ | N | |
| 254 | $CH_2C\equiv CH$ | 6-Cl | H | $OCH_3$ | $OCH_3$ | CH | |
| 255 | " | 6-Cl | H | $OCH_3$ | $CH_3$ | CH | |
| 256 | " | 6-Cl | H | $OCH_3$ | $CH_3$ | N | |
| 257 | $CH_2CH_2OCH_3$ | 6-Cl | H | $OCH_3$ | $OCH_3$ | CH | |
| 258 | " | 6-Cl | H | $OCH_3$ | $CH_3$ | CH | |
| 259 | " | 6-Cl | H | $OCH_3$ | $CH_3$ | N | |
| 260 | $CH_2CCH_3=CH_2$ | 6-Cl | H | $OCH_3$ | $OCH_3$ | CH | |
| 261 | " | 6-Cl | H | $OCH_3$ | $CH_3$ | CH | |
| 262 | " | 6-Cl | H | $OCH_3$ | $CH_3$ | N | |
| 263 | $C_6H_5$ | 6-Cl | H | $OCH_3$ | $OCH_3$ | CH | |
| 264 | " | 6-Cl | H | $OCH_3$ | $CH_3$ | CH | |
| 265 | " | 6-Cl | H | $OCH_3$ | $CH_3$ | N | |
| 266 | $CH_2CH(OCH_3)_2$ | 6-Cl | H | $OCH_3$ | $OCH_3$ | CH | |
| 267 | " | 6-Cl | H | $OCH_3$ | $CH_3$ | CH | |
| 268 | " | 6-Cl | H | $OCH_3$ | $CH_3$ | N | |
| 269 | $CH_2CH=CH_2$ | 5-Cl | H | $OCH_3$ | $OCH_3$ | CH | |
| 270 | " | 5-Cl | H | $OCH_3$ | $CH_3$ | CH | |
| 271 | $CH_2CH_2Cl$ | 5-Cl | H | $OCH_3$ | $CH_3$ | N | |

| Bsp.Nr. | $R^1$ | $(R^2)$ | $R^3$ | $R^5$ | $R^6$ | E | Fp. |
|---|---|---|---|---|---|---|---|
| 272 | $CH_2CH_2Cl$ | 5-Cl | H | $OCH_3$ | $OCH_3$ | CH | |
| 273 | $CH_2CH_2OCH_3$ | 5-Cl | H | $OCH_3$ | $OCH_3$ | CH | |
| 274 | " | 5-Cl | H | $OCH_3$ | $CH_3$ | N | |
| 275 | $CH_2C≡CH$ | 5-Cl | H | $OCH_3$ | $OCH_3$ | CH | |
| 276 | " | 5-Cl | H | $OCH_3$ | $CH_3$ | CH | |
| 277 | $CH_2CH=CHCl$ | 4-Cl | H | $OCH_3$ | $CH_3$ | N | |
| 278 | " | 4-Cl | H | $OCH_3$ | $OCH_3$ | CH | |
| 279 | $CH_2C_6H_5$ | 3-Cl | H | $OCH_3$ | $OCH_3$ | CH | |
| 280 | " | 3-Cl | H | $OCH_3$ | $CH_3$ | N | |
| 281 | $CH_2CH=CH_2$ | 6-F | H | $OCH_3$ | $OCH_3$ | CH | |
| 282 | " | 6-F | H | $OCH_3$ | $CH_3$ | CH | |
| 283 | " | 6-F | H | $OCH_3$ | $CH_3$ | N | |
| 284 | $CH_2H_2Cl$ | 6-F | H | $OCH_3$ | $OCH_3$ | CH | |
| 285 | " | 6-F | H | $OCH_3$ | $CH_3$ | CH | |
| 286 | " | 6-F | H | $OCH_3$ | $CH_3$ | N | |
| 287 | $CH_2C≡CH$ | 6-F | H | $OCH_3$ | $OCH_3$ | CH | |
| 288 | " | 6-F | H | $OCH_3$ | $CH_3$ | CH | |
| 289 | " | 6-F | H | $OCH_3$ | $CH_3$ | N | |
| 290 | $CH_2CH_2OCH_3$ | 6-F | H | $OCH_3$ | $OCH_3$ | CH | |
| 291 | " | 6-F | H | $OCH_3$ | $CH_3$ | CH | |
| 292 | " | 6-F | H | $OCH_3$ | $CH_3$ | N | |
| 293 | $CH_2CH=CHCH_3$ | 6-F | H | OCH | $OCH_3$ | CH | |
| 294 | " | 6-F | H | $OCH_3$ | $CH_3$ | CH | |
| 295 | " | 6-F | H | $OCH_3$ | $CH_3$ | N | |
| 296 | $CH_2C≡CCH_3$ | 6-F | H | OCH | OCH | CH | |
| 297 | " | 6-F | H | $OCH_3$ | $CH_3$ | CH | |
| 298 | " | 6-F | H | $OCH_3$ | $CH_3$ | N | |
| 299 | $CH_2CH=CH_2$ | 5-F | H | $OCH_3$ | $OCH_3$ | CH | |

| Bsp.Nr. | $R^1$ | $(R^2)_n$ | $R^3$ | $R^5$ | $R^6$ | E | Fp. |
|---|---|---|---|---|---|---|---|
| 300 | $CH_2CH=CH_2$ | 5-F | H | $OCH_3$ | $CH_3$ | CH | |
| 301 | $CH_2CH_2Cl$ | 5-F | H | $OCH_3$ | $CH_3$ | N | |
| 302 | " | 5-F | H | $OCH_3$ | $OCH_3$ | CH | |
| 303 | $CH_2CH_2OCH_3$ | 5-F | H | $OCH_3$ | $OCH_3$ | CH | |
| 304 | " | 5-F | H | $OCH_3$ | $CH_3$ | N | |
| 305 | $CH_2C≡CH$ | 5-F | H | $OCH_3$ | $OCH_3$ | CH | |
| 306 | " | 5-F | H | $OCH_3$ | $CH_3$ | CH | |
| 307 | $CH_2CH_2Br$ | 4-F | H | $OCH_3$ | $CH_3$ | N | |
| 308 | " | 4-F | H | $OCH_3$ | $OCH_3$ | CH | |
| 309 | $CH_2CH=CH_2$ | 6-$COOCH_3$ | H | $OCH_3$ | $OCH_3$ | CH | |
| 310 | " | 6-$COOCH_3$ | H | $OCH_3$ | $CH_3$ | CH | |
| 311 | " | 6-$COOCH_3$ | H | $OCH_3$ | $CH_3$ | N | |
| 312 | $CH_2CH_2Cl$ | 6-$COOCH_3$ | H | $OCH_3$ | $OCH_3$ | CH | |
| 313 | " | 6-$COOCH_3$ | H | $OCH_3$ | $CH_3$ | CH | |
| 314 | " | 6-$COOCH_3$ | H | $OCH_3$ | $CH_3$ | N | |
| 315 | $CH_2C≡CH$ | 6-$COOCH_3$ | H | $OCH_3$ | $OCH_3$ | CH | |
| 316 | " | 6-$COOCH_3$ | H | $OCH_3$ | $CH_3$ | CH | |
| 317 | " | 6-$COOCH_3$ | H | $OCH_3$ | $CH_3$ | N | |
| 318 | $CH_2CH_2OCH_3$ | 6-$COOCH_3$ | H | $OCH_3$ | $OCH_3$ | CH | |
| 319 | " | 6-$COOCH_3$ | H | $OCH_3$ | $CH_3$ | CH | |
| 320 | " | 6-$COOCH_3$ | H | $OCH_3$ | $CH_3$ | N | |
| 321 | $CH_2CH=CHCH_3$ | 6-$COOCH_3$ | H | $OCH_3$ | $OCH_3$ | CH | |
| 322 | " | 6-$COOCH_3$ | H | $OCH_3$ | $CH_3$ | CH | |
| 323 | " | 6-$COOCH_3$ | H | $OCH_3$ | $CH_3$ | N | |
| 324 | $CH_2CH=CHCl$ | 6-$COOCH_3$ | H | $OCH_3$ | $OCH_3$ | CH | |
| 325 | " | 6-$COOCH_3$ | H | $OCH_3$ | $CH_3$ | CH | |
| 326 | " | 6-$COOCH_3$ | H | $OCH_3$ | $CH_3$ | N | |
| 327 | $C_6H_5$ | 6-$COOCH_3$ | H | $OCH_3$ | $OCH_3$ | CH | |
| 328 | " | 6-$COOCH_3$ | H | $OCH_3$ | $CH_3$ | CH | |
| 329 | " | 6-$COOCH_3$ | H | $OCH_3$ | $CH_3$ | N | |
| 330 | $CH_2CH=CH_2$ | 5-$COOCH_3$ | H | $OCH_3$ | $OCH_3$ | CH | |
| 331 | " | 5-$COOCH_3$ | H | $OCH_3$ | $CH_3$ | CH | |
| 332 | $CH_2CH_2Cl$ | 5-$COOCH_3$ | H | $OCH_3$ | $CH_3$ | N | |

| Bsp.Nr. | $R^1$ | $(R^2)_n$ | $R^3$ | $R^5$ | $R^6$ | E | Fp. |
|---|---|---|---|---|---|---|---|
| 333 | $CH_2CH_2Cl$ | 5-$COOCH_3$ | H | $OCH_3$ | $OCH_3$ | CH | |
| 334 | $CH_2CH_2OCH_3$ | 5-$COOCH_3$ | H | $OCH_3$ | $OCH_3$ | CH | |
| 335 | " | 5-$COOCH_3$ | H | $OCH_3$ | $CH_3$ | N | |
| 336 | $CH_2C{\equiv}CH$ | 5-$COOCH_3$ | H | $OCH_3$ | $OCH_3$ | CH | |
| 337 | " | 5-$COOCH_3$ | H | $OCH_3$ | $CH_3$ | CH | |
| 338 | $CH_2CH_2Br$ | 4-$COOCH_3$ | H | $OCH_3$ | $CH_3$ | N | |
| 339 | " | 4-$COOCH_3$ | H | $OCH_3$ | $OCH_3$ | CH | |
| 340 | $CH_2CCl{=}CH_2$ | 3-$COOCH_3$ | H | $OCH_3$ | $OCH_3$ | CH | |
| 341 | " | 3-$COOCH_3$ | H | $OCH_3$ | $CH_3$ | N | |
| 342 | $CH_2CH{=}CH_2$ | 6-$CH_2CH{=}CH_2$ | H | $OCH_3$ | $OCH_3$ | CH | |
| 343 | " | 6-$CH_2CH{=}CH_2$ | H | $OCH_3$ | $CH_3$ | CH | |
| 344 | " | 6-$CH_2CH{=}CH_2$ | H | $OCH_3$ | $CH_3$ | N | |
| 345 | $CH_2CH_2Cl$ | 6-$CH_2CH{=}CH_2$ | H | $OCH_3$ | $OCH_3$ | CH | |
| 346 | " | 6-$CH_2CH{=}CH_2$ | H | $OCH_3$ | $CH_3$ | CH | |
| 347 | " | 6-$CH_2CH{=}CH_2$ | H | $OCH_3$ | $CH_3$ | N | |
| 348 | $CH_2C{\equiv}CH$ | 6-$CH_2CH{=}CH_2$ | H | $OCH_3$ | $OCH_3$ | CH | |
| 349 | " | 6-$CH_2CH{=}CH_2$ | H | $OCH_3$ | $CH_3$ | CH | |
| 350 | " | 6-$CH_2CH{=}CH_2$ | H | $OCH_3$ | $CH_3$ | N | |
| 351 | $CH_2CH_2OCH_3$ | 6-$CH_2CH{=}CH_2$ | H | $OCH_3$ | $OCH_3$ | CH | |
| 352 | " | 6-$CH_2CH{=}CH_2$ | H | $OCH_3$ | $CH_3$ | CH | |
| 353 | " | 6-$CH_2CH{=}CH_2$ | H | $OCH_3$ | $CH_3$ | N | |
| 354 | $CH_2CH_3$ | 6-$CH_2CH{=}CH_2$ | H | $OCH_3$ | $OCH_3$ | CH | 131-132 |
| 355 | " | 6-$CH_2CH{=}CH_2$ | H | $OCH_3$ | $CH_3$ | CH | 134-136 |
| 356 | " | 6-$CH_2CH{=}CH_2$ | H | $OCH_3$ | $CH_3$ | N | |
| 357 | $CH(CH_3)_2$ | 6-$CH_2CH{=}CH_2$ | H | $OCH_3$ | $OCH_3$ | CH | |
| 358 | " | 6-$CH_2CH{=}CH_2$ | H | $OCH_3$ | $CH_3$ | CH | |
| 359 | " | 6-$CH_2CH{=}CH_2$ | H | $OCH_3$ | $CH_3$ | N | |
| 360 | $CH_2CH_2CH_3$ | 6-$CH_2CH{=}CH_2$ | H | $OCH_3$ | $OCH_3$ | CH | |
| 361 | " | 6-$CH_2CH{=}CH_2$ | H | $OCH_3$ | $CH_3$ | CH | |
| 362 | " | 6-$CH_2CH{=}CH_2$ | H | $OCH_3$ | $CH_3$ | N | |
| 363 | $CH_2CH{=}CH_2$ | 6-$CH_2CH_3$ | H | $OCH_3$ | $OCH_3$ | CH | |
| 364 | " | 6-$CH_2CH_3$ | H | $OCH_3$ | $CH_3$ | CH | |
| 365 | $CH_2CH_2Cl$ | 6-$CH_2CH_3$ | H | $OCH_3$ | $CH_3$ | N | |
| 366 | " | 6-$CH_2CH_3$ | H | $OCH_3$ | $OCH_3$ | CH | |
| 367 | $CH_2CH_2OCH_3$ | 6-$CH_2CH_3$ | H | $OCH_3$ | $OCH_3$ | CH | |

| Bsp.Nr. | $R^1$ | $(R^2)_n$ | $R^3$ | $R^5$ | $R^6$ | E | Fp. |
|---|---|---|---|---|---|---|---|
| 368 | $CH_2CH_2OCH_3$ | 6-$CH_2CH_3$ | H | $OCH_3$ | $CH_3$ | N | |
| 369 | $CH_2C{\equiv}CH$ | 6-$CH_2CH_3$ | H | $OCH_3$ | $OCH_3$ | CH | |
| 370 | " | 6-$CH_2CH_3$ | H | $OCH_3$ | $CH_3$ | CH | |
| 371 | $CH_2CH_2Br$ | 6-$CH_2CH_3$ | H | $OCH_3$ | $CH_3$ | N | |
| 372 | " | 6-$CH_2CH_3$ | H | $OCH_3$ | $OCH_3$ | CH | |
| 373 | $CH_2CCl{=}CH_2$ | 6-$CH_2CH_3$ | H | $OCH_3$ | OCH | CH | |
| 374 | " | 6-$CH_2CH_3$ | H | $OCH_3$ | $CH_3$ | N | |
| 375 | $CH_2CH{=}CH_2$ | 6-$OCH_3$ | H | $OCH_3$ | $OCH_3$ | CH | |
| 376 | " | 6-$OCH_3$ | H | $OCH_3$ | $CH_3$ | CH | |
| 377 | $CH_2CH_2Cl$ | 6-$OCH_3$ | H | $OCH_3$ | $CH_3$ | N | |
| 378 | " | 6-$OCH_3$ | H | $OCH_3$ | $OCH_3$ | CH | |
| 379 | $CH_2CH_2OCH_3$ | 6-$OCH_3$ | H | $OCH_3$ | $OCH_3$ | CH | |
| 380 | " | 6-$OCH_3$ | H | $OCH_3$ | $CH_3$ | N | |
| 381 | $CH_2C{\equiv}CH$ | 6-$OCH_3$ | H | $OCH_3$ | $OCH_3$ | CH | |
| 382 | " | 6-$OCH_3$ | H | $OCH_3$ | $CH_3$ | CH | |
| 383 | $CH_2CH_2Cl$ | 6-$OC_6H_5$ | H | $OCH_3$ | $CH_3$ | N | |
| 384 | " | 6-$OC_6H_5$ | H | $OCH_3$ | $OCH_3$ | CH | |
| 385 | $CH_2CH{=}CH_2$ | 6-$OC_6H_5$ | H | $OCH_3$ | $OCH_3$ | CH | |
| 386 | " | 6-$OC_6H_5$ | H | $OCH_3$ | $CH_3$ | N | |
| 387 | $CH_2C{\equiv}CH$ | 6-$OC_6H_5$ | H | $OCH_3$ | $OCH_3$ | CH | |
| 388 | " | 6-$OC_6H_5$ | H | $OCH_3$ | $CH_3$ | CH | |
| 389 | $CH_2CH_2OCH_3$ | 6-$OC_6H_5$ | H | $OCH_3$ | $CH_3$ | N | |
| 390 | " | 6-$OC_6H_5$ | H | $OCH_3$ | $OCH_3$ | CH | |
| 391 | " | 6-$C_6H_5$ | H | $OCH_3$ | $OCH_3$ | CH | |
| 392 | " | 6-$C_6H_5$ | H | $OCH_3$ | $CH_3$ | N | |
| 393 | $CH_2C{\equiv}CH$ | 6-$C_6H_5$ | H | $OCH_3$ | $OCH_3$ | CH | |
| 394 | " | 6-$C_6H_5$ | H | $OCH_3$ | $CH_3$ | CH | |
| 395 | $CH_2CH_2Br$ | 6-$SO_2CH_3$ | H | $OCH_3$ | $CH_3$ | N | |
| 396 | " | 6-$SO_2CH_3$ | H | $OCH_3$ | $OCH_3$ | CH | |
| 397 | $CH_2CCl{=}CH_2$ | 6-$SO_2CH_3$ | H | $OCH_3$ | $OCH_3$ | CH | |
| 398 | " | 6-$SO_2CH_3$ | H | $OCH_3$ | $CH_3$ | N | |
| 399 | $CH_2CH{=}CH_2$ | 6-$SOCH_3$ | H | $OCH_3$ | $OCH_3$ | CH | |
| 400 | " | 6-$SOCH_3$ | H | $OCH_3$ | $CH_3$ | CH | |
| 401 | $CH_2CH_2Cl$ | 6-$SOCH_3$ | H | $OCH_3$ | $CH_3$ | N | |
| 402 | " | 6-$SOCH_3$ | H | $OCH_3$ | $OCH_3$ | CH | |

| Bsp.Nr. | $R^1$ | $(R^2)_n$ | $R^3$ | $R^5$ | $R^6$ | E | Fp. |
|---|---|---|---|---|---|---|---|
| 403 | $CH_2CH_2OCH_3$ | 6-$SCH_3$ | H | $OCH_3$ | $OCH_3$ | CH | |
| 404 | " | 6-$SCH_3$ | H | $OCH_3$ | $CH_3$ | N | |
| 405 | $CH_2C\equiv CH$ | 6-$SCH_3$ | H | $OCH_3$ | $OCH_3$ | CH | |
| 406 | " | 6-$SCH_3$ | H | $OCH_3$ | $CH_3$ | CH | |
| 407 | $CH_2CH_2Br$ | 6-$CF_3$ | H | $OCH_3$ | $CH_3$ | N | |
| 408 | " | 6-$CF_3$ | H | $OCH_3$ | $OCH_3$ | CH | |
| 409 | $CH_2CCl{=}CH_2$ | 6-$NO_2$ | H | $OCH_3$ | $OCH_3$ | CH | |
| 410 | $CH_2CH_2Cl$ | 4,6-$Cl_2$ | H | $OCH_3$ | Cl | CH | |
| 411 | " | 4,6-$Cl_2$ | H | $OCH_3$ | $OCH_3$ | | |
| 412 | " | 4,6-$Cl_2$ | H | $OCH_3$ | $CH_3$ | N | |
| 413 | " | 3,5-$Cl_2$ | H | $OCH_3$ | $OCH_3$ | CH | |
| 414 | $CH_2CH_2OCH_3$ | 4,6-$Cl_2$ | H | $OCH_3$ | Cl | CH | |
| 415 | " | 4,6-$F_2$ | H | $OCH_3$ | $OCH_3$ | CH | |
| 416 | " | 4,6-$F_2$ | H | $OCH_3$ | $CH_3$ | CH | |
| 417 | " | 3,5-$F_2$ | H | $OCH_3$ | $OCH_3$ | CH | |
| 418 | $CH_2CH{=}CH_2$ | 4,6-$F_2$ | H | $OCH_3$ | $CH_3$ | N | |
| 419 | " | 4,6-$Cl_2$ | H | $OCH_3$ | $OCH_3$ | CH | |
| 420 | " | 4,6-$Cl_2$ | H | $OCH_3$ | Cl | CH | |
| 421 | " | 3,5-$Cl_2$ | H | $OCH_3$ | $OCH_3$ | CH | |
| 422 | $CH_2C\equiv CH$ | 4,6-$Cl_2$ | H | $OCF_2H$ | $CH_3$ | CH | |
| 423 | " | 4,6-$F_2$ | H | $OCH_3$ | $OCH_3$ | CH | |
| 424 | " | 4,6-$F_2$ | H | $OCH_3$ | $CH_3$ | N | |
| 425 | " | 3,5-$F_2$ | H | $OCH_3$ | $OCH_3$ | CH | |
| 426 | $C_6H_5$ | 4,6-$F_2$ | H | $OCH_3$ | Cl | CH | |
| 427 | " | 4,6-$Cl_2$ | H | $OCH_3$ | $OCH_3$ | CH | |
| 428 | $CH_2C_6H_5$ | 4,6-$Cl_2$ | H | $OCF_2H$ | $OCF_2H$ | CH | |
| 429 | " | 4,6-$F_2$ | H | $OCH_3$ | $OCH_3$ | CH | |
| 430 | $CH_2CH{=}CHCH_3$ | 4,6-$F_2$ | H | $OCH_3$ | $CH_3$ | N | |
| 431 | " | 4,6-$Cl_2$ | H | $OCH_3$ | $OCH_3$ | CH | |
| 432 | $CH_2CH{=}CHCl$ | 4,6-$Cl_2$ | H | $OCH_3$ | Cl | CH | |
| 433 | " | 4,6-$(CH_3)_2$ | H | $OCH_3$ | $OCH_3$ | CH | |
| 434 | " | 4,6-$(NO_2)_2$ | H | $OCH_2CF_3$ | $OCH_3$ | CH | |
| 435 | $CH_2CH_2Cl$ | H | $CH_3$ | $OCH_3$ | $OCH_3$ | CH | |
| 436 | " | H | $CH_3$ | $OCH_3$ | $CH_3$ | N | |
| 437 | " | H | $CH_2CH{=}CH_2$ | $OCH_3$ | $OCH_3$ | CH | |
| 438 | " | H | $CH_2CH{=}CH_2$ | $OCH_3$ | Cl | CH | |

| Bsp.Nr. | $R^1$ | $(R^2)_n$ | $R^3$ | $R^5$ | $R^6$ | E | Fp. |
|---|---|---|---|---|---|---|---|
| 439 | $CH_2CH_2OCH_3$ | H | $CH_3$ | $OCH_3$ | $OCH_3$ | CH | |
| 440 | " | H | $CH_3$ | $OCH_3$ | $CH_3$ | CH | |
| 441 | " | H | $CH_2CH{=}CH_2$ | $OCH_3$ | $OCH_3$ | CH | |
| 442 | " | H | $CH_2CH{=}CH_2$ | $OCH_3$ | $CH_3$ | N | |
| 443 | $CH_2CH{=}CH_2$ | H | $CH_3$ | $OCH_3$ | $OCH_3$ | CH | |
| 444 | " | H | $CH_3$ | $OCH_3$ | Cl | CH | |
| 445 | " | H | $CH_2CH{=}CH_2$ | $OCH_3$ | $OCH_3$ | CH | |
| 446 | " | H | $CH_2CH{=}CH_2$ | $OCF_2H$ | $CH_3$. | CH | |
| 447 | $CH_2C{\equiv}CH$ | H | $CH_3$ | $OCH_3$ | $OCH_3$ | CH | |
| 448 | " | H | $CH_3$ | $OCH_3$ | $CH_3$ | N | |
| 449 | " | H | $CH_2CH{=}CH_2$ | $OCH_3$ | $OCH_3$ | CH | |
| 450 | " | H | $CH_2CH{=}CH_2$ | $OCH_3$ | Cl | CH | |
| 451 | $CH_2C{\equiv}CCH_3$ | H | $CH_3$ | $OCH_3$ | $OCH_3$ | CH | |
| 452 | " | H | $CH_2CH{=}CH_2$ | $OCF_2H$ | $OCF_2H$ | CH | |
| 453 | $CH_2CH{=}CHCl$ | H | $CH_2CH{=}CH_2$ | $OCH_3$ | $OCH_3$ | CH | |
| 454 | " | H | $CH_3$ | $OCH_3$ | $CH_3$ | N | |
| 455 | $C_6H_5$ | H | $C_2H_5$ | $OCH_3$ | $OCH_3$ | CH | |
| 456 | $CH_2CH_2Br$ | H | $C_2H_5$ | $OCH_3$ | Cl | CH | |
| 457 | $CH_2CCH_3{=}CH_2$ | H | $C_2H_5$ | $OCH_3$ | $OCH_3$ | CH | |
| 458 | $CH_2C{\equiv}CH$ | H | $C_2H_5$ | $OCH_3$ | $OCH_3$ | CH | |
| 459 | $CH_2C_6H_5$ | 6-$CH_3$ | $CH_3$ | $OCH_3$ | $CH_3$ | N | |
| 460 | $CH_2CH_2OCH_3$ | 6-$OCH_3$ | $CH_3$ | $OCH_3$ | $OCH_3$ | CH | |
| 461 | $CH_2CH{=}CH_2$ | 6-Cl | $CH_2CH{=}CH_2$ | $OCH_3$ | Cl | CH | |
| 462 | $H_2CH(OCH_3)_2$ | 6-F | $CH_2CH{=}CH_2$ | $OCH_3$ | $OCH_3$ | CH | |
| 463 | $CH_2C{\equiv}CH$ | 6-$CF_3$ | $CH_3$ | $OCH_2CF_3$ | $OCH_3$ | N | |

**Tabelle 2**

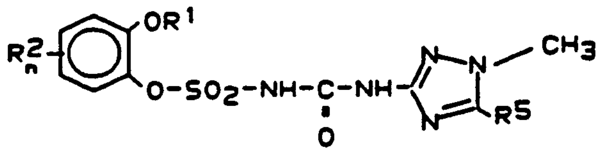

| Bsp.Nr. | $R^1$ | $(R^2)_n$ | $R^5$ | Fp. |
|---|---|---|---|---|
| 664 | $CH_2CH{=}CH_2$ | H | $CH_3$ | |
| 665 | " | H | H | |
| 666 | " | H | $OCH_3$ | |
| 667 | $CH_2CH_2Cl$ | H | $OCH_3$ | |
| 668 | $CH_2CH_2OCH_3$ | H | $OCH_3$ | |
| 669 | $CH_2C{\equiv}CH$ | H | $OCH_3$ | |
| 670 | $CH_2C_6H_5$ | H | $OCH_3$ | |
| 671 | $CH_2CH{=}CHCl$ | H | $OCH_3$ | |
| 672 | $CH_2CH{=}CHCH_3$ | H | $OCH_3$ | |
| 673 | $CH_2CH_2Br$ | H | $OCH_3$ | |
| 674 | $CH_2C{\equiv}CCH_3$ | H | $OCH_3$ | |
| 675 | $CH_2CH{=}CH_2$ | 6-$CH_3$ | $OCH_3$ | |
| 676 | $CH_2CH_2Cl$ | 6-$OCH_3$ | $OCH_3$ | |
| 677 | $CH_2CH_2OCH_3$ | 6-Cl | $OCH_3$ | |
| 678 | $CH_2CH{=}CH_2$ | 6-$CF_3$ | $OCH_3$ | |
| 679 | $CH_2C{\equiv}CH$ | 6-F | $OCH_3$ | |
| 680 | $CH_2CH{=}CHCH_3$ | 6-$OCF_2H$ | $OCH_3$ | |

**Tabelle 3**

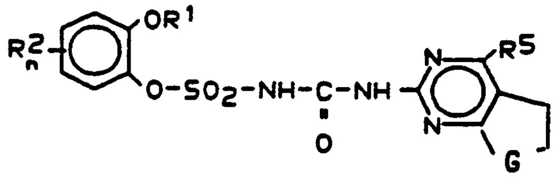

| Bsp.Nr. | $R^1$ | $R^2$ | $R^5$ | G | n | Fp.[°C] |
|---|---|---|---|---|---|---|
| 481 | $CH_2CH=CH_2$ | H | $CH_3$ | $CH_2$ | 0 | |
| 482 | " | H | H | $CH_2$ | 0 | |
| 483 | " | H | $OCH_3$ | $CH_2$ | 0 | |
| 484 | $CH_2CH_2Cl$ | H | $CH_3$ | O | 0 | |
| 485 | $CH_2CH_2OCH_3$ | H | $CH_3$ | $CH_2$ | 0 | |
| 486 | $CH_2C{\equiv}CH$ | H | $CH_3$ | O | 0 | |
| 487 | $CH_2C_6H_5$ | H | $CH_3$ | $CH_2$ | 0 | |
| 488 | $CH_2CH=CHCl$ | H | $CH_3$ | O | 0 | |
| 489 | $CH_2CH=CCH_3$ | H | $CH_3$ | $CH_2$ | 0 | |
| 490 | $CH_2CH_2Br$ | H | $CH_3$ | O | 0 | |
| 491 | $CH_2C{\equiv}CCH_3$ | H | $CH_3$ | $CH_2$ | 0 | |
| 492 | $CH_2CH=CH_2$ | 6-$CH_3$ | $CH_3$ | $CH_2$ | 1 | |
| 493 | $CH_2CH_2Cl$ | 6-$OCH_3$ | $CH_3$ | O | 1 | |
| 494 | $CH_2CH_2OCH_3$ | 6-Cl | $CH_3$ | $CH_2$ | 1 | |
| 495 | $CH_2CH=CH_2$ | 6-$CF_3$ | $CH_3$ | O | 1 | |
| 496 | $CH_2C{\equiv}CH$ | 6-F | $CH_3$ | $CH_2$ | 1 | |
| 497 | $CH_2CH=CHCH_3$ | 6-$OCF_2H$ | $CH_3$ | O | 1 | |

**Tabelle 4**

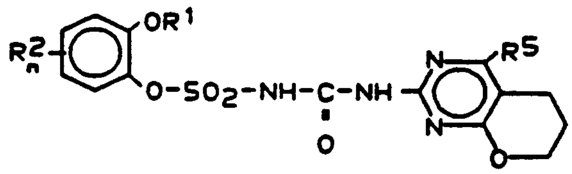

| Bsp.Nr. | $R^1$ | $R^2$ | $R^5$ | n | Fp. |
|---|---|---|---|---|---|
| 498 | $CH_2CH=CH_2$ | H | $CH_3$ | 0 | |
| 499 | " | H | H | 0 | |
| 500 | " | H | $OCH_3$ | 0 | |
| 501 | $CH_2CH_2Cl$ | H | $CH_3$ | 0 | |
| 502 | $CH_2CH_2OCH_3$ | H | $CH_3$ | 0 | |
| 503 | $CH_2C\equiv CH$ | H | $CH_3$ | 0 | |
| 504 | $CH_2C_6H_5$ | H | $CH_3$ | 0 | |
| 505 | $CH_2CH=CHCl$ | H | $CH_3$ | 0 | |
| 506 | $CH_2CH=CHCH_3$ | H | $CH_3$ | 0 | |
| 507 | $CH_2CH_2Br$ | H | $CH_3$ | 0 | |
| 508 | $CH_2C\equiv CCH_3$ | H | $CH_3$ | 0 | |
| 509 | $CH_2CH=CH_2$ | 6-$CH_3$ | $CH_3$ | 1 | |
| 510 | $CH_2CH_2Cl$ | 6-$OCH_3$ | $CH_3$ | 1 | |
| 511 | $CH_2CH_2OCH_3$ | 6-Cl | $CH_3$ | 1 | |
| 512 | $CH_2CH=CH_2$ | 6-F | $CH_3$ | 1 | |
| 513 | $CH_2C\equiv CH$ | 6-F | $CH_3$ | 1 | |
| 514 | $CH_2CH=CHCH_3$ | 6-$OCF_2H$ | $CH_3$ | 1 | |

**Tabelle 5**

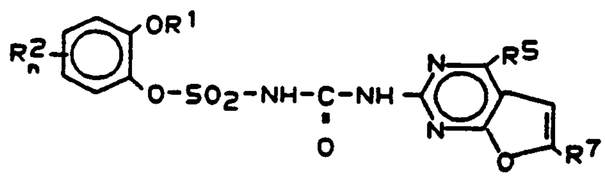

| Bsp.Nr. | $R^1$ | $R^2$ | $R^5$ | $R^7$ | n | Fp. |
|---|---|---|---|---|---|---|
| 515 | $CH_2CH=CH_2$ | H | $CH_3$ | $CH_3$ | 0 | |
| 516 | " | H | H | $CH_3$ | 0 | |
| 517 | " | H | $OCH_3$ | $CH_3$ | 0 | |
| 518 | $CH_2CH_2Cl$ | H | $CH_3$ | $CH_3$ | 0 | |
| 519 | $CH_2CH_2OCH_3$ | H | $CH_3$ | H | 0 | |
| 520 | $CH_2C\equiv CH$ | H | $CH_3$ | $CH_3$ | 0 | |
| 521 | $CH_2C_6H_5$ | H | $CH_3$ | H | 0 | |
| 522 | $CH_2CH=CHCl$ | H | $CH_3$ | $CH_3$ | 0 | |
| 523 | $CH_2CH=CHCH_3$ | H | $CH_3$ | H | 0 | |
| 524 | $CH_2CH_2Br$ | H | $CH_3$ | $CH_3$ | 0 | |
| 525 | $CH_2C\equiv CCH_3$ | H | $CH_3$ | H | 0 | |
| 526 | $CH_2CH=CH_2$ | 6-$CH_3$ | $CH_3$ | H | 1 | |
| 527 | $CH_2CH_2Cl$ | 6-$OCH_3$ | $CH_3$ | $CH_3$ | 1 | |
| 528 | $CH_2CH_2OCH_3$ | 6-Cl | $CH_3$ | H | 1 | |
| 529 | $CH_2CH=CH_2$ | 6-$CF_3$ | $CH_3$ | $CH_3$ | 1 | |
| 530 | $CH_2C\equiv CH$ | 6-F | $CH_3$ | H | 1 | |
| 531 | $CH_2CH=CHCH_3$ | 6-$OCF_2H$ | $CH_3$ | $CH_3$ | 1 | |

**Tabelle 6**

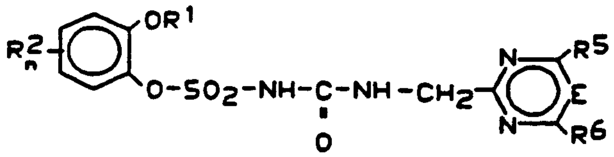

| Bsp.Nr. | $R^1$ | $R^2$ | $R^5$ | $R^6$ | E | n | Fp. |
|---|---|---|---|---|---|---|---|
| 532 | $CH_2CH=CH_2$ | H | $OCH_3$ | $OCH_3$ | CH | 0 | |
| 533 | " | H | $OCH_3$ | $CH_3$ | CH | 0 | |
| 534 | " | H | $OCH_3$ | $CH_3$ | N | 0 | |
| 535 | $CH_2CH_2Cl$ | H | $OCH_3$ | $CH_3$ | CH | 0 | |
| 536 | $CH_2CH_2OCH_3$ | H | $OCH_3$ | $CH_3$ | N | 0 | |
| 537 | $CH_2C{\equiv}CH$ | H | $OCH_3$ | $OCH_3$ | CH | 0 | |
| 538 | $CH_2C_6H_5$ | H | $OCH_3$ | $CH_3$ | N | 0 | |
| 539 | $CH_2CH=CHCl$ | H | $OCH_3$ | $CH_3$ | CH | 0 | |
| 540 | $CH_2CH=CHCH_3$ | H | $OCH_3$ | $OCH_3$ | N | 0 | |
| 541 | $CH_2CH_2Br$ | H | $OCH_3$ | $CH_3$ | CH | 0 | |
| 542 | $CH_2C{\equiv}CCH_3$ | H | $OCH_3$ | $CH_3$ | N | 0 | |
| 543 | $CH_2CH=CH_2$ | 6-$CH_3$ | $OCH_3$ | $CH_3$ | N | 1 | |
| 544 | $CH_2CH_2Cl$ | 6-$OCH_3$ | $OCH_3$ | $CH_3$ | CH | 1 | |
| 545 | $CH_2CH_2OCH_3$ | 6-Cl | $OCH_3$ | $OCH_3$ | N | 1 | |
| 546 | $CH_2CH=CH_2$ | 6-$CF_3$ | $OCH_3$ | $CH_3$ | CH | 1 | |
| 547 | $CH_2C{\equiv}CH$ | 6-F | $OCH_3$ | $OCH_3$ | N | 1 | |
| 548 | $CH_2CH=CHCH_3$ | 6-$OCF_2H$ | $OCH_3$ | $CH_3$ | CH | 1 | |

**Tabelle 7**

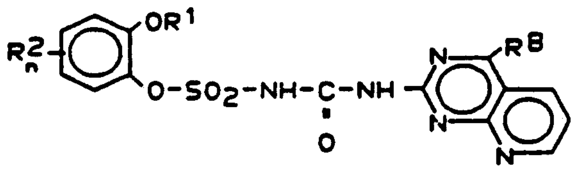

| Bsp.Nr. | $R^1$ | $R^2$ | $R^8$ | n | Fp. |
|---|---|---|---|---|---|
| 549 | $CH_2CH=CH_2$ | H | $OCH_3$ | 0 | |
| 550 | " | H | $CH_3$ | 0 | |
| 551 | " | 3-Cl | $CH_3$ | 0 | |
| 552 | $CH_2CH_2Cl$ | H | $CH_3$ | 0 | |
| 553 | $CH_2CH_2OCH_3$ | H | $CH_3$ | 0 | |
| 554 | $CH_2C{\equiv}CH$ | H | $OCH_3$ | 0 | |
| 555 | $CH_2CH=CHCl$ | H | $CH_3$ | 0 | |
| 556 | $CH_2CH=CHCl$ | H | $CH_3$ | 0 | |
| 557 | $CH_2CH=CHCH_3$ | H | $OCH_3$ | 0 | |
| 558 | $CH_2CH_2Br$ | H | $CH_3$ | 0 | |
| 559 | $CH_2C{\equiv}CCH_3$ | H | $CH_3$ | 0 | |
| 560 | $CH_2CH=CH_2$ | 6-$CH_3$ | $CH_3$ | 1 | |
| 561 | $CH_2CH_2Cl$ | 6-$OCH_3$ | $CH_3$ | 1 | |
| 562 | $CH_2CH_2OCH_3$ | 6-Cl | $OCH_3$ | 1 | |
| 563 | $CH_2CH=CH_2$ | 6-$CF_3$ | $CH_3$ | 1 | |
| 564 | $CH_2C{\equiv}CH$ | 6-F | $OCH_3$ | 1 | |
| 565 | $CH_2CH=CHCH_3$ | 6-$OCF_2H$ | $CH_3$ | 1 | |

**Tabelle 8**

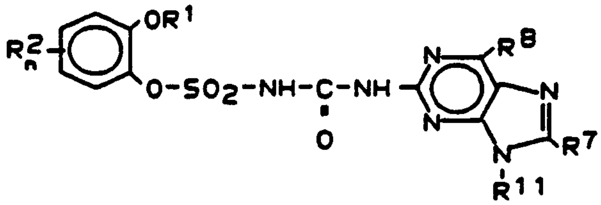

| Bsp.Nr. | $R^1$ | $R^2$ | R7 | $R^8$ | $R^{11}$ | n | Fp. |
|---|---|---|---|---|---|---|---|
| 566 | $CH_2CH=CH_2$ | H | H | $OCH_3$ | H | 0 | |
| 567 | " | H | H | $CH_3$ | $CH_3$ | 0 | |
| 568 | " | H | $CH_3$ | $CH_3$ | $CH_3$ | 0 | |
| 569 | $CH_2CH_2Cl$ | H | H | $CH_3$ | H | 0 | |
| 570 | $CH_2CH_2OCH_3$ | H | H | $CH_3$ | $CH_3$ | 0 | |
| 571 | $CH_2C≡CH$ | H | $CH_3$ | $OCH_3$ | $CHF_2$ | 0 | |
| 572 | $CH_2C_6H_5$ | H | H | $CH_3$ | H | 0 | |
| 573 | $CH_2CH=CHCl$ | H | H | $CH_3$ | $CH_3$ | 0 | |
| 574 | $CH_2CH=CHCH_3$ | H | $CH_3$ | $OCH_3$ | $CH_3$ | 0 | |
| 575 | $CH_2CH_2Br$ | H | H | $CH_3$ | H | 0 | |
| 576 | $CH_2C≡CCH_3$ | H | H | $CH_3$ | $CH_2CF_3$ | 0 | |
| 577 | $CH_2CH=CH_2$ | 6-$CH_3$ | H | $CH_3$ | H | 1 | |
| 578 | $CH_2CH_2Cl$ | 6-OCH3 | H | $CH_3$ | $CH_3$ | 1 | |
| 579 | $CH_2CH_2OCH_3$ | 6-Cl | $CH_3$ | $OCH_3$ | $CH_3$ | 1 | |
| 580 | $CH_2CH=CH_2$ | 6-$CF_3$ | H | $CH_3$ | H | 1 | |
| 581 | $CH_2C≡CH$ | 6-F | H | $OCH_3$ | $CH_3$ | 1 | |
| 582 | $CH_2CH=CHCH_3$ | 6-Cl | $CH_3$ | $CH_3$ | $CH_3$ | 1 | |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet:

0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100 % Wirkung bzw. Schaden

**1. Unkrautwirkung im Vorauflauf**

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte in Tabelle 9 zeigen, weisen die erfindungsgemäßen Verbindungen eine gute herbizide Vorauflaufwirksamkeit gegen ein breites Spektrum von Ungräsern und Unkräutern auf.

**2. Unkrautwirkung im Nachauflauf**

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 10).

**3. Kulturpflanzenverträglichkeit**

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel I weisen somit eine hohe Selektivität bei Anwendung zur Bekämpfung von unerwünschtem Pflanzenwuchs in landwirtschaftlichen Kulturen auf.

Tabelle 9

| Vorauflaufwirkung der erfindungsgemäßen Verbindungen | | | | | | | |
|---|---|---|---|---|---|---|---|
| Bsp. Nr. | Dosierung kg a.i./ha | herbizide Wirkung | | | | | |
| Nr. | | SIA | CRS | STM | AS | ECG | LOM |
| 2 | 0,6 | 5 | 5 | 5 | 1 | 4 | 3 |
| 3 | 0,6 | 5 | 5 | 5 | 2 | 5 | 2 |
| 4 | 0,6 | 5 | 5 | 5 | 1 | 3 | 2 |
| 71 | 0,6 | 5 | 5 | 3 | 3 | 4 | 4 |
| 74 | 0,6 | 5 | 5 | 4 | 1 | 3 | 1 |
| 84 | 0,6 | 5 | 5 | 5 | 1 | 3 | 1 |

Tabelle 10

| Nachlaufwirkung der erfindungsgemäßen Verbindungen | | | | | | | |
|---|---|---|---|---|---|---|---|
| Bsp. Nr. | Dosierung kg a.i./ha | herbizide Wirkung | | | | | |
| Nr. | | SIA | CRS | STM | AS | ECG | LOM |
| 2 | 0,6 | 5 | 5 | 5 | 1 | 4 | 3 |
| 3 | 0,6 | 5 | 5 | 5 | 1 | 4 | 2 |
| 4 | 0,6 | 5 | 4 | 4 | 1 | 4 | 2 |
| 71 | 0,6 | 5 | 4 | 4 | 3 | 4 | 3 |
| 74 | 0,6 | 5 | 5 | 5 | 1 | 3 | 2 |
| 84 | 0,6 | 5 | 5 | 5 | 0 | 5 | 3 |

Abkürzungen:

SIA = Sinapis alba
CRS = Chrysanthemum segetum
STM = Stellaria media
AS = Avena sativa
ECG = Echinochloa crus-galli
LOM = Lolium multiflorum
a.i. = Aktivsubstanz

**Patentansprüche**
**Patentansprüche für folgende Vertragsstaaten : BE, CH, DE, FR, GB, IT, LI, NL, SE**

**1.** Verbindungen der Formel I oder deren Salze

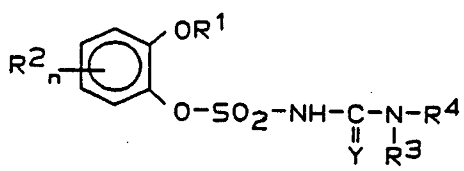

(I),

worin

$R^1$ $(C_1\text{-}C_8)$Alkyl, $(C_2\text{-}C_8)$Alkenyl, $(C_2\text{-}C_8)$Alkinyl, wobei diese Reste jeweils ein- oder mehrfach durch Chlor oder Brom ein- oder zweifach durch $(C_1\text{-}C_6)$Alkoxy, $(C_2\text{-}C_6)$Alkenyloxy, $(C_2\text{-}C_6)$Alkinyloxy, $(C_1\text{-}C_6)$Alkylthio, $(C_1\text{-}C_6)$Alkylsulfinyl, $(C_1\text{-}C_6)$Alkylsulfonyl, $(C_1\text{-}C_6)$-Alkoxycarbonyl, $NO_2$, CN oder durch Phenyl substituiert sind; ferner $(C_2\text{-}C_8)$Alkenyl, $(C_2\text{-}C_8)$Alkinyl, $(C_3\text{-}C_8)$Cycloalkyl, das ein- oder mehrfach durch Halogen oder ein- oder zweifach durch $(C_1\text{-}C_4)$Alkoxy oder $(C_1\text{-}C_4)$Alkylthio substituiert sein kann; $(C_5\text{-}C_8)$-Cycloalkenyl, Cyclopropylmethyl, Epoxypropyl; Furfuryl, Tetrahydrofurfuryl, Phenoxy$(C_1\text{-}C_4)$alkyl, wobei die drei letztgenannten Substituenten durch Halogen, $(C_1\text{-}C_4)$Alkyl, $(C_1\text{-}C_4)$Alkoxy substituiert sein können; oder Phenyl, das ein- oder mehrfach durch Halogen, $(C_1\text{-}C_4)$Alkyl, $(C_1\text{-}C_4)$Alkoxy, $NO_2$, $CF_3$, CN oder $OCHF_2$ substituiert sein kann;
oder für den Fall, daß $R^2$ $(C_2\text{-}C_8)$Alkenyl, $(C_2\text{-}C_8)$Alkinyl, Phenyl, Phenoxy, die wie unten angegeben substituiert sein können, $(C_1\text{-}C_4)$Alkylsulfonyl oder $(C_1\text{-}C_4)$Alkylsulfinyl bedeutet und $n \neq 0$ ist, $R^1$ auch $(C_1\text{-}C_8)$Alkyl bedeutet,

$R^2$ unabhängig voneinander $(C_1\text{-}C_8)$Alkyl, $(C_2\text{-}C_8)$-Alkenyl, $(C_2\text{-}C_8)$Alkinyl, Phenyl, Phenoxy, $(C_1\text{-}C_4)$Alkoxy, $(C_1\text{-}C_4)$-Alkylthio, $(C_1\text{-}C_4)$Alkoxycarbonyl, $(C_2\text{-}C_4)$Alkenyloxycarbonyl, $(C_2\text{-}C_4)$Alkinyloxycarbonyl, wobei alle vorgenannten Reste ein- oder mehrfach durch Halogen oder ein- bis zweifach durch $(C_1\text{-}C_4)$Alkoxy oder $(C_1\text{-}C_4)$-Alkylthio substituiert sein können; ferner Halogen, $NO_2$, $(C_1\text{-}C_4)$-Alkylsulfonyl oder $(C_1\text{-}C_4)$Alkylsulfinyl;

n 0, 1, 2 oder 3;

y O oder S;

$R^3$ Wasserstoff, $(C_1\text{-}C_8)$Alkyl, $(C_2\text{-}C_8)$Alkenyl, $(C_3\text{-}C_8)$Alkinyl oder $(C_1\text{-}C_4)$Alkoxy;

$R^4$ einen heterocyclischen Rest der Formeln

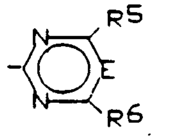 , 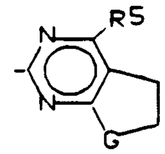 , 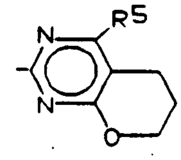 ,

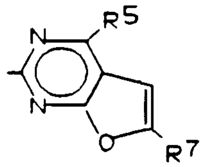 , 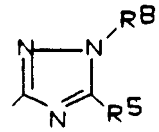 , 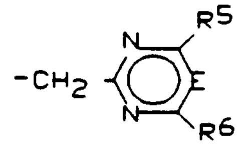 ,

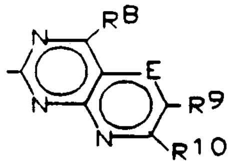 oder 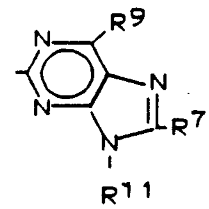

E CH oder N,

G O oder $CH_2$,

$R^5$, $R^6$ unabhängig voneinander Wasserstoff, Halogen, $(C_1\text{-}C_6)$-Alkyl, $(C_1\text{-}C_6)$Alkoxy oder $(C_1\text{-}C_6)$Alkylthio, wobei die vorgenannten alkylhaltigen Reste ein- oder mehrfach durch Halogen oder ein- oder zweifach durch $(C_1\text{-}C_4)$Alkoxy oder $(C_1\text{-}C_4)$Alkylthio substituiert sein können, ferner einen Rest der Formel $-NR^{12}R^{13}$, $-OCHR^7\text{-}CO_2R^{12}$, $(C_3\text{-}C_6)$-Cycloalkyl, $(C_3\text{-}C_5)$Alkenyl, $(C_2\text{-}C_4)$Alkinyl, $(C_3\text{-}C_5)$- Alkenyloxy oder $(C_3\text{-}C_5)$Alkinyloxy,

$R^7$ Wasserstoff oder $(C_1\text{-}C_4)$Alkyl,

$R^8$ $(C_1\text{-}C_4)$Alkyl, $-CHF_2$ oder $-CH_2CF_3$,

$R^9$, $R^{10}$ unabhängig voneinander Wasserstoff, $(C_1\text{-}C_4)$Alkyl, $(C_1\text{-}C_5)$Alkoxy oder Halogen,

$R^{11}$ Wasserstoff, $(C_1\text{-}C_4)$Alkyl, $-CHF_2$ oder $CH_2CF_3$ und

$R^{12}$, $R^{13}$ unabhängig voneinander Waserstoff, $(C_1\text{-}C_4)$Alkyl, $(C_2\text{-}C_4)$Alkenyl oder $(C_3\text{-}C_4)$Alkinyl

bedeuten.

**2.** Verbindungen der Formel I von Anspruch 1 oder deren Salze, worin $R^1$ $(C_1\text{-}C_4)$Alkyl, $(C_2\text{-}C_5)$Alkenyl oder $(C_2\text{-}C_4)$Alkinyl, wobei diese Reste wie in Anspruch 1 angegeben substituiert sind; ferner $(C_2\text{-}C_5)$-Alkenyl oder $(C_2\text{-}C_4)$Alkinyl oder für den Fall, daß n = 1 und $R^2$ = $(C_2\text{-}C_8)$Alkenyl oder $(C_2\text{-}C_8)$Alkinyl ist, einen Rest $(C_1\text{-}C_4)$Alkyl; $R^2$ $(C_1\text{-}C_4)$-Alkyl, $(C_2\text{-}C_5)$-Alkenyl, $(C_2\text{-}C_4)$Alkinyl, $(C_1\text{-}C_4)$Alkoxycarbonyl, Phenyl, Phenoxy, $(C_1\text{-}C_4)$Alkoxy, $(C_1\text{-}C_4)$Alkylthio, wobei diese Reste wie in Anspruch 1 angegebene substituiert sein können; oder Halogen; n 0, 1 oder 2, Y = O, $R^3$ Wasserstoff, $(C_1\text{-}C_4)$Alkyl oder $(C_3\text{-}C_4)$Alkenyl, $R^4$ einen heterocyclischen Rest der Formel

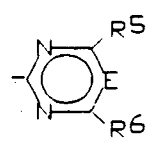 ,

E = CH oder N ,

$R^5$ und $R^6$ unabhängig voneinander Halogen, $(C_1\text{-}C_4)$Alkyl, $(C_1\text{-}C_4)$-Alkoxy, $(C_1\text{-}C_4)$Alkylthio, die wie in Anspruch 1 angegeben substituiert sein können, bedeuten.

**3.** Verbindungen der Formel I von Anspruch 1 oder deren Salze, worin $R^1$ $(C_1\text{-}C_4)$Alkyl, $(C_2\text{-}C_5)$Alkenyl, $(C_2\text{-}C_4)$Alkinyl, wobei diese Reste wie in Anspruch 1 angegeben substituiert sind, ferner $(C_2\text{-}C_5)$Alkenyl oder $(C_2\text{-}C_4)$Alkinyl $R^2$ $(C_1\text{-}C_4)$Alkyl, $(C_2\text{-}C_5)$Alkenyl, $(C_1\text{-}C_4)$-Alkoxycarbonyl, $(C_1\text{-}C_4)$Alkoxy, $(C_1\text{-}C_4)$-Alkylthio, die wie in Anspruch 1 angegeben substituiert sein können; Fluor oder Chlor, n = 0 oder 1, $R^3$ Wasserstoff oder Methyl, $R^4$ einen heterocyclischen Rest der Formel

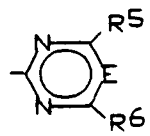

E = CH oder N
und $R^5$ und $R^6$ unabhängig voneinander Chlor, Brom, $(C_1\text{-}C_4)$Alkyl, $(C_1\text{-}C_4)$Alkoxy, $OCHF_2$, $OCH_2CF_3$ oder $CF_3$ bedeuten.

**4.** Verfahren zur Herstellung der Verbindungen der Formel I von Ansprüchen 1 bis 3 oder deren Salze, dadurch gekennzeichnet, daß man
(a) eine Verbindung der Formel (II)

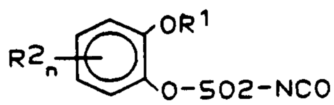

(II)

mit einer Verbindung der Formel (III)

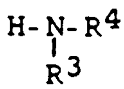

(III)

umsetzt, oder
(b) eine Verbindung der Formel (IV)

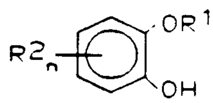

(IV)

mit einem Chlorsulfonylharnstoff der Formel (V)

$$Cl\text{-}SO2\text{-}NH\text{-}CO\text{-}\underset{\displaystyle R^3}{\underset{|}{N}}\text{-}R^4 \qquad (V)$$

umsetzt, oder
(c) eine Verbindung der Formel (VI)

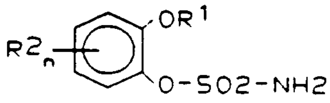

(VI)

mit einem Carbamat der Formel (VII)

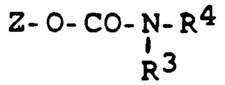

(VII)

worin Z Phenyl oder $(C_1-C_6)$Alkyl bedeutet, umsetzt, und die erhaltenen Verbindungen der Formel I gegebenfalls in ihre Salze überführt.

**5.** Herbizide oder pflanzenwachstumsregulierende Mittel, dadurch gekennzeichnet, daß sie eine wirksame Menge einer oder mehrerer Verbindungen der Formel I, oder deren Salze gemäß einem der Ansprüche 1 bis 3 und inerte Hilfsstoffe enthalten.

**6.** Verwendung der Verbindungen der Formel I oder deren Salze gemäß Ansprüche 1 bis 3 als Herbizide oder Pflanzenwachstumsregulatoren.

**7.** Verfahren zur Bekämpfung von Schadpflanzen, dadurch gekennzeichnet, daß man auf diese oder die landwirtschaftlich oder industriell genutzten Böden eine wirksame Menge einer oder mehrerer Verbindungen der Formel I oder deren Salz gemäß einem der Ansprüche 1 bis 3 appliziert.

**8.** Verfahren zur Wachstumsregulierung von Nutzpflanzen, dadurch gekennzeichnet, daß man auf diese oder die Anbaufläche eine wirksame Menge einer oder mehrerer Verbindungen der Formel I oder deren Salze gemäß einem der Ansprüche 1 bis 3 appliziert.

**Patentansprüche für folgenden Vertragsstaat : ES**

**1.** Verfahren zur Bekämpfung von Schadpflanzen, dadurch gekennzeichnet, daß man auf diese oder die landwirtschaftlich oder industriell genutzten Böden eine wirksame Menge einer oder mehrerer Verbindungen der Formel I oder deren Salze

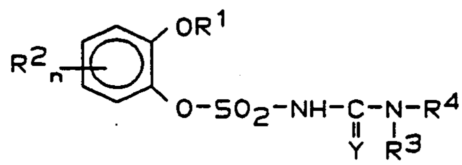

(I),

worin

| | |
|---|---|
| $R^1$ | $(C_1-C_8)$Alkyl, $(C_2-C_8)$Alkenyl, $(C_2-C_8)$Alkinyl, wobei diese Reste jeweils ein- oder mehrfach durch Chlor oder Brom ein- oder zweifach durch $(C_1-C_6)$Alkoxy, $(C_2-C_6)$Alkenyloxy, $(C_2-C_6)$Alkinyloxy, $(C_1-C_6)$Alkylthio, $(C_1-C_6)$Alkylsulfinyl, $(C_1-C_6)$Alkylsulfonyl, $(C_1-C_6)$-Alkoxycarbonyl, $NO_2$, CN oder durch Phenyl substituiert sind; ferner $(C_2-C_8)$Alkenyl, $(C_2-C_8)$Alkinyl, $(C_3-C_8)$Cycloalkyl, das ein- oder mehrfach durch Halogen oder ein- oder zweifach durch $(C_1-C_4)$Alkoxy oder $(C_1-C_4)$Alkylthio substituiert sein kann; $(C_5-C_8)$-Cycloalkenyl, Cyclopropylmethyl, Epoxypropyl; Furfuryl, Tetrahydrofurfuryl, Phenoxy$(C_1-C_4)$alkyl, wobei die drei letztgenannten Substituenten durch Halogen, $(C_1-C_4)$Alkyl, $(C_1-C_4)$Alkoxy substituiert sein können; oder Phenyl, das ein- oder mehrfach durch Halogen, $(C_1-C_4)$Alkyl, $(C_1-C_4)$Alkoxy, $NO_2$, $CF_3$, CN oder $OCHF_2$ substituiert sein kann; oder für den Fall, daß $R^2$ $(C_2-C_8)$Alkenyl, $(C_2-C_8)$Alkinyl, Phenyl, Phenoxy, die wie unten angegeben substituiert sein können, $(C_1-C_4)$Alkylsulfonyl oder $(C_1-C_4)$Alkylsulfinyl bedeutet und $n \neq 0$ ist, $R^1$ auch $(C_1-C_8)$Alkyl bedeutet, |
| $R^2$ | unabhängig voneinander $(C_1-C_8)$Alkyl, $(C_2-C_8)$-Alkenyl, $(C_2-C_8)$Alkinyl, Phenyl, Phenoxy, $(C_1-C_4)$Alkoxy, $(C_1-C_4)$-Alkylthio, $(C_1-C_4)$Alkoxycarbonyl, $(C_2-C_4)$Alkenyloxycarbonyl, $(C_2-C_4)$Alkinyloxycarbonyl, wobei alle vorgenannten Reste ein- oder mehrfach durch Halogen, oder ein- bis zweifach durch $(C_1-C_4)$Alkoxy oder $(C_1-C_4)$-Alkylthio substituiert sein können; ferner Halogen, $NO_2$, $(C_1-C_4)$-Alkylsulfonyl oder $(C_1-C_4)$Alkylsulfinyl; |
| n | 0, 1, 2 oder 3; |
| y | O oder S; |

| | |
|---|---|
| $R^3$ | Wasserstoff, $(C_1$-$C_8)$Alkyl, $(C_2$-$C_8)$Alkenyl, $(C_3$-$C_8)$Alkinyl oder $(C_1$-$C_4)$Alkoxy; |
| $R^4$ | einen heterocyclischen Rest der Formeln |

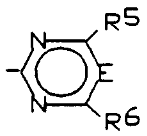
,
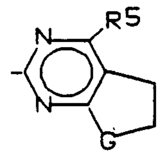
,
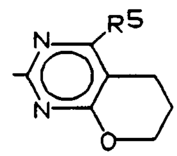
,
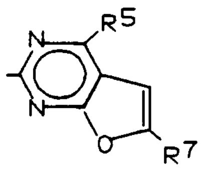
,
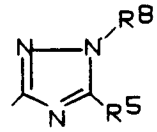
,
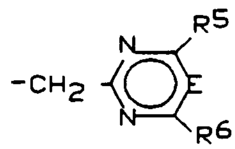
,
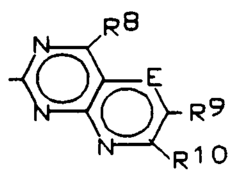
oder
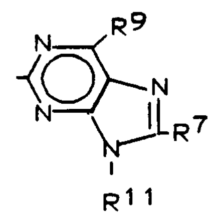

| | |
|---|---|
| E | CH oder N, |
| G | O oder $CH_2$, |
| $R^5$, $R^6$ | unabhängig voneinander Wasserstoff, Halogen, $(C_1$-$C_6)$-Alkyl, $(C_1$-$C_6)$Alkoxy oder $(C_1$-$C_6)$Alkylthio, wobei die vorgenannten alkylhaltigen Reste ein- oder mehrfach durch Halogen oder ein- oder zweifach durch $(C_1$-$C_4)$Alkoxy oder $(C_1$-$C_4)$Alkylthio substituiert sein können, ferner einen Rest der Formel $-NR^{12}R^{13}$, $-OCHR^7-CO_2R^{12}$, $(C_3$-$C_6)$-Cycloalkyl, $(C_3$-$C_5)$Alkenyl, $(C_2$-$C_4)$Alkinyl, $(C_3$-$C_5)$- Alkenyloxy oder $(C_3$-$C_5)$Alkinyloxy, |
| $R^7$ | Wasserstoff oder $(C_1$-$C_4)$Alkyl, |
| $R^8$ | $(C_1$-$C_4)$Alkyl, $-CHF_2$ oder $-CH_2CF_3$, |
| $R^9$, $R^{10}$ | unabhängig voneinander Wasserstoff, $(C_1$-$C_4)$Alkyl, $(C_1$-$C_5)$Alkoxy oder Halogen, |
| $R^{11}$ | Wasserstoff, $(C_1$-$C_4)$Alkyl, $-CHF_2$ oder $CH_2CF_3$ und |
| $R^{12}$, $R^{13}$ | unabhängig voneinander Waserstoff, $(C_1$-$C_4)$Alkyl, $(C_2$-$C_4)$Alkenyl oder $(C_3$-$C_4)$Alkinyl |

bedeuten, appliziert.

**2.** Verfahren nach Anspruch 1, dadurch gekennzeichnet, daß in Formel I $R^1$ $(C_1$-$C_4)$Alkyl, $(C_2$-$C_5)$Alkenyl oder $(C_2$-$C_4)$Alkinyl, wobei diese Reste wie in Anspruch 1 angegeben substituiert sind; ferner $(C_2$-$C_5)$-Alkenyl oder $(C_2$-$C_4)$Alkinyl oder für den Fall, daß n = 1 und $R^2$ = $(C_2$-$C_8)$Alkenyl oder $(C_2$-$C_8)$Alkinyl ist, einen Rest $(C_1$-$C_4)$Alkyl; $R^2$ $(C_1$-$C_4)$-Alkyl, $(C_2$-$C_5)$-Alkenyl, $(C_2$-$C_4)$Alkinyl, $(C_1$-$C_4)$Alkoxycarbonyl, Phenyl, Phenoxy, $(C_1$-$C_4)$Alkoxy, $(C_1$-$C_4)$Alkylthio, wobei diese Reste wie in Anspruch 1 angegebene substituiert sein können; oder Halogen; n 0, 1 oder 2, Y = O, $R^3$ Wasserstoff, $(C_1$-$C_4)$Alkyl oder $(C_3$-$C_4)$Alkenyl, $R^4$ einen heterocyclischen Rest der Formel

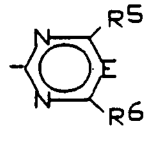
,

E = CH oder N ,
$R^5$ und $R^6$ unabhängig voneinander Halogen, $(C_1$-$C_4)$Alkyl, $(C_1$-$C_4)$-Alkoxy, $(C_1$-$C_4)$Alkylthio, die wie in Anspruch 1 angegeben substituiert sein können, bedeuten.

**3.** Verfahren nach Anspruch 1 oder 2, dadurch gekennzeichnet, daß in Formel I $R^1$ $(C_1$-$C_4)$Alkyl, $(C_2$-$C_5)$-Alkenyl, $(C_2$-$C_4)$-Alkinyl, wobei diese Reste wie in Anspruch 1 angegeben substituiert sind, ferner $(C_2$-$C_5)$Alkenyl oder $(C_2$-$C_4)$Alkinyl $R^2$ $(C_1$-$C_4)$Alkyl, $(C_2$-$C_5)$Alkenyl, $C_1$-$C_4)$-Alkoxycarbonyl, $(C_1$-$C_4)$Alkoxy, $(C_1$-$C_4)$Alkylthio, die wie in Anspruch 1 angegeben substituiert sein können; Fluor oder Chlor, n = 0 oder 1, $R^3$ Wasserstoff oder Methyl, $R^4$ einen heterocyclischen Rest der Formel

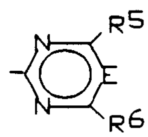

E = CH oder N
und $R^5$ und $R^6$ unabhängig voneinander Chlor, Brom, $(C_1$-$C_4)$Alkyl, $(C_1$-$C_4)$Alkoxy, $OCHF_2$, $OCH_2CF_3$ oder $CF_3$ bedeuten.

**4.** Verfahren zur Herstellung der Verbindungen der in Anspruch 1, 2 oder 3 definierten Formel I oder deren Salze, dadurch gekennzeichnet, daß man
(a) eine Verbindung der Formel (II)

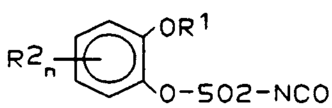

(II)

mit einer Verbindung der Formel (III)

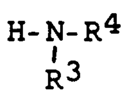

(III)

umsetzt, oder
(b) eine Verbindung der Formel (IV)

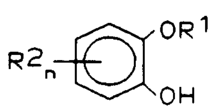

(IV)

mit einem Chlorsulfonylharnstoff der Formel (V)

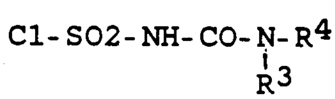

(V)

umsetzt, oder

(c) eine Verbindung der Formel (VI)

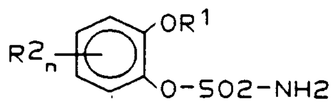

(VI)

mit einem Carbamat der Formel (VII)

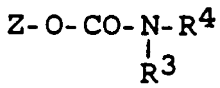

(VII)

worin, Z Phenyl oder $(C_1\text{-}C_6)$Alkyl bedeutet, umsetzt, und die erhaltenen Verbindungen der Formel I gegebenfalls in ihre Salze überführt.

**5.** Verfahren nach Anspruch 1, 2 oder 3, dadurch gekennzeichnet, daß zwischen 0,005 und 10 kg/ha Verbindung der Formel I appliziert wird.

**6.** Verwendung der Verbindungen der in Anspruch 1, 2 oder 3 definierten Formel I oder deren Salze als Herbizide oder Pflanzenwachstumsregulatoren.

**7.** Verfahren nach Anspruch 5, dadurch gekennzeichnet, daß man zwischen 0,01 und 5 kg/ha appliziert.

**8.** Verfahren zur Wachstumsregulierung von Nutzpflanzen, dadurch gekennzeichnet, daß man auf diese oder die Anbaufläche eine wirksame Menge einer oder mehrerer Verbindungen der in Anspruch 1, 2 oder 3 definierten Formel I oder deren Salze appliziert.

**Claims**
**Claims for the following Contracting States : BE, CH, DE, FR, GB, IT, LI, NL, SE**

**1.** A compound of the formula I or a salt thereof

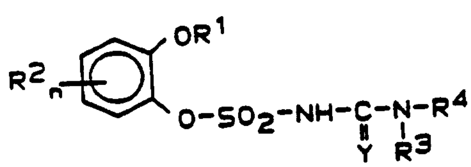

(I),

where

$R^1$ is $(C_1\text{-}C_8)$alkyl, $(C_2\text{-}C_8)$alkenyl, $(C_2\text{-}C_8)$alkynyl, each of these radicals being monosubstituted or polysubstituted by chlorine or bromine or monosubstituted or disubstituted by $(C_1\text{-}C_6)$alkoxy, $(C_2\text{-}C_6)$alkenyloxy, $(C_2\text{-}C_6)$alkynyloxy, $(C_1\text{-}C_6)$alkylthio, $(C_1\text{-}C_6)$alkylsulfinyl, $(C_1\text{-}C_6)$alkylsulfonyl, $(C_1\text{-}C_6)$alkoxycarbonyl, $NO_2$, CN or by phenyl; furthermore $(C_2\text{-}C_8)$alkenyl, $(C_2\text{-}C_8)$alkynyl, $(C_3\text{-}C_8)$cycloalkyl, which can be monosubstituted or polysubstituted by halogen or monosubstituted or disubstituted by $(C_1\text{-}C_4)$alkoxy or $(C_1\text{-}C_4)$alkylthio; $(C_5\text{-}C_8)$cycloalkenyl, cyclopropylmethyl, epoxypropyl; furfuryl, tetrahydrofurfuryl, phenoxy-$(C_1\text{-}C_4)$alkyl, it being possible for the last three substituents mentioned to be substituted by halogen, $(C_1\text{-}C_4)$alkyl or $(C_1\text{-}C_4)$alkoxy; or phenyl which can be monosubstituted or polysubstituted by halogen, $(C_1\text{-}C_4)$alkyl, $(C_1\text{-}C_4)$alkoxy, $NO_2$, $CF_3$, CN or $OCHF_2$; or in the event that $R^2$ is $(C_2\text{-}C_8)$alkenyl, $(C_2\text{-}C_8)$alkynyl, phenyl, phenoxy, each of which can be substituted as indicated below, $(C_1\text{-}C_4)$alkylsulfonyl or $(C_1\text{-}C_4)$alkylsulfinyl and $n \neq 0$, $R^1$ is also $(C_1\text{-}C_8)$alkyl,

$R^2$ radicals independently of one another are $(C_1\text{-}C_8)$alkyl, $(C_2\text{-}C_8)$alkenyl, $(C_2\text{-}C_8)$-

alkynyl, phenyl, phenoxy, $(C_1-C_4)$alkoxy, $(C_1-C_4)$alkylthio, $(C_1-C_4)$alkoxycarbonyl, $(C_2-C_4)$alkenyloxycarbonyl or $(C_2-C_4)$alkynyloxycarbonyl, it being possible for all of the abovementioned radicals to be monosubstituted or polysubstituted by halogen or monosubstituted to disubstituted by $(C_1-C_4)$alkoxy or $(C_1-C_4)$alkylthio; furthermore are halogen, $NO_2$, $(C_1-C_4)$alkylsulfonyl or $(C_1-C_4)$alkylsulfinyl;

n is 0, 1, 2 or 3;

Y is O or S;

$R^3$ is hydrogen, $(C_1-C_8)$alkyl, $(C_2-C_8)$alkenyl, $(C_3-C_8)$alkynyl or $(C_1-C_4)$alkoxy;

$R^4$ is a heterocyclic radical of the formula

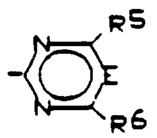 , 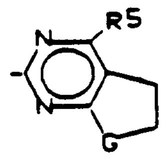 , 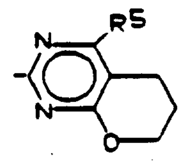 ,

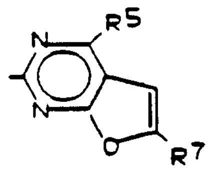 , 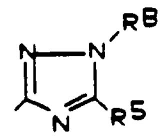 , 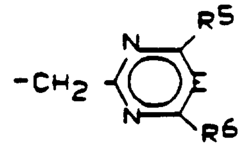 ,

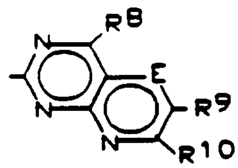 or 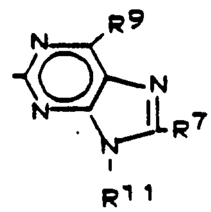

E is CH or N,

G is O or $CH_2$,

$R^5$ and $R^6$ independently of one another are hydrogen, halogen, $(C_1-C_6)$alkyl, $(C_1-C_6)$alkoxy or $(C_1-C_6)$alkylthio, it being possible for the abovementioned alkyl-containing radicals to be monosubstituted or polysubstituted by halogen or monosubstituted or disubstituted by $(C_1-C_4)$alkoxy or $(C_1-C_4)$alkylthio, furthermore are a radical of the formula $-NR^{12}R^{13}$, $-OCHR^7-CO_2R^{12}$, $(C_3-C_6)$cycloalkyl, $(C_3-C_5)$alkenyl, $(C_2-C_4)$alkynyl, $(C_3-C_5)$alkenyloxy or $(C_3-C_5)$alkynyloxy,

$R^7$ is hydrogen or $(C_1-C_4)$alkyl,

$R^8$ is $(C_1-C_4)$alkyl, $-CHF_2$ or $-CH_2CF_3$,

$R^9$ and $R^{10}$ independently of one another are hydrogen, $(C_1-C_4)$alkyl, $(C_1-C_5)$alkoxy or halogen,

$R^{11}$ is hydrogen, $(C_1-C_4)$alkyl, $-CHF_2$ or $CH_2CF_3$ and

$R^{12}$ and $R^{13}$ independently of one another are hydrogen, $(C_1-C_4)$alkyl, $(C_2-C_4)$alkenyl or $(C_3-C_4)$-alkynyl.

**2.** A compound of the formula I of claim 1 or a salt thereof, where $R^1$ is $(C_1-C_4)$alkyl, $(C_2-C_5)$alkenyl or $(C_2-C_4)$alkynyl, these radicals being substituted as indicated in claim 1; furthermore $(C_2-C_5)$alkenyl or $(C_2-C_4)$alkynyl or, in the event that n = 1 and $R^2$ is $(C_2-C_8)$alkenyl or $(C_2-C_8)$alkynyl, is a $(C_1-C_4)$alkyl radical; $R^2$ is $(C_1-C_4)$alkyl, $(C_2-C_5)$alkenyl, $(C_2-C_4)$alkynyl, $(C_1-C_4)$alkoxycarbonyl, phenyl, phenoxy, $(C_1-C_4)$alkoxy, $(C_1-C_4)$alkylthio, it being possible for these radicals to be substituted as indicated in claim 1; or is halogen; n is 0, 1 or 2, Y is O, $R^3$ is hydrogen, $(C_1-C_4)$alkyl or $(C_3-C_4)$alkenyl, $R^4$ is a heterocyclic radical of the formula

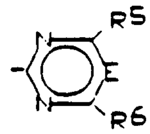 ,

E is CH or N,
$R^5$ and $R^6$ independently of one another are halogen, $(C_1\text{-}C_4)$alkyl, $(C_1\text{-}C_4)$alkoxy or $(C_1\text{-}C_4)$alkylthio, each of which can be substituted as indicated in claim 1.

**3.** A compound of the formula I of claim 1 or a salt thereof, where $R^1$ is $(C_1\text{-}C_4)$alkyl, $(C_2\text{-}C_5)$alkenyl, $(C_2\text{-}C_4)$alkynyl, these radicals being substituted as indicated in claim 1, furthermore $(C_2\text{-}C_5)$alkenyl or $(C_2\text{-}C_4)$alkynyl, $R^2$ is $(C_1\text{-}C_4)$alkyl, $(C_2\text{-}C_5)$alkenyl, $(C_1\text{-}C_4)$alkoxycarbonyl, $(C_1\text{-}C_4)$alkoxy or $(C_1\text{-}C_4)$alkylthio, each of which can be substituted as indicated in claim 1; or is fluorine or chlorine, n = 0 or 1, $R^3$ is hydrogen or methyl, $R^4$ is a heterocyclic radical of the formula

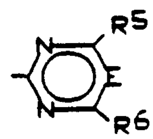

E = CH or N
and $R^5$ and $R^6$ independently of one another are chlorine, bromine, $(C_1\text{-}C_4)$alkyl, $(C_1\text{-}C_4)$alkoxy, $OCHF_2$, $OCH_2CF_3$ or $CF_3$.

**4.** A process for the preparation of a compound of the formula I of any one of claims 1 to 3 or a salt thereof, which comprises reacting
(a) a compound of the formula (II)

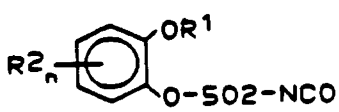

(II)

with a compound of the formula (III)

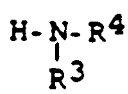

(III),

or
(b) a compound of the formula (IV)

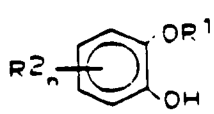

(IV)

with a chlorosulfonylurea of the formula (V)

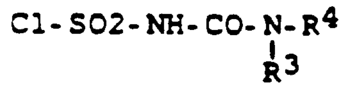

(V),

or

(c) a compound of the formula (VI)

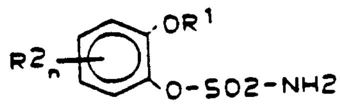

(VI)

with a carbamate of the formula (VII)

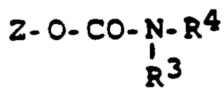

(VII)

where Z is phenyl or $(C_1\text{-}C_6)$alkyl, and, if desired, converting the resultant compound of the formula I into a salt thereof.

**5.** A herbicidal or plant growth-regulating agent, containing an effective amount of one or more compounds of the formula I or a salt thereof as claimed in any one of claims 1 to 3 and inert auxiliaries.

**6.** The use of a compound of the formula I or a salt thereof as claimed in any one of claims 1 to 3 as a herbicide or plant growth regulator.

**7.** A method of controlling weed plants, which comprises applying an effective amount of one or more compounds of the formula I or a salt thereof, as claimed in any one of claims 1 to 3, to these weed plants or the soil used for agriculture or industry.

**8.** A method of regulating the growth of crop plants, which comprises applying an effective amount of one or more compounds of the formula I or a salt thereof, as claimed in any one of claims 1 to 3, to these crop plants or the cropped area.

**Claims for the following Contracting State : ES**

**1.** A method of controlling weed plants, which comprises applying to these weed plants or the soil used for agriculture or industry an effective amount of one or more compounds of the formula I.

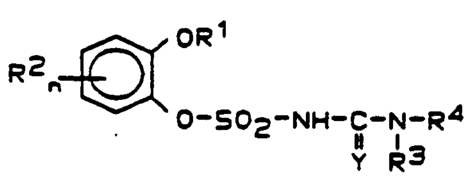

(I),

where

$R^1$ is $(C_1\text{-}C_8)$alkyl, $(C_2\text{-}C_8)$alkenyl, $(C_2\text{-}C_8)$alkynyl, each of these radicals being monosubstituted or polysubstituted by chlorine or bromine or monosubstituted or disubstituted by $(C_1\text{-}C_6)$alkoxy, $(C_2\text{-}C_6)$alkenyloxy, $(C_2\text{-}C_6)$alkynyloxy, $(C_1\text{-}C_6)$alkylthio, $(C_1\text{-}C_6)$alkylsulfinyl, $(C_1\text{-}C_6)$alkylsulfonyl, $(C_1\text{-}C_6)$alkoxycarbonyl, $NO_2$, CN or by phe-

nyl; furthermore ($C_2$-$C_8$)alkenyl, ($C_2$-$C_8$)alkynyl, ($C_3$-$C_8$)cycloalkyl, which can be monosubstituted or polysubstituted by halogen or monosubstituted or disubstituted by ($C_1$-$C_4$)alkoxy or ($C_1$-$C_4$)alkylthio; ($C_5$-$C_8$)cycloalkenyl, cyclopropylmethyl, epoxypropyl; furfuryl, tetrahydrofurfuryl, phenoxy-($C_1$-$C_4$)alkyl, it being possible for the last three substituents mentioned to be substituted by halogen, ($C_1$-$C_4$)alkyl or ($C_1$-$C_4$)alkoxy; or phenyl which can be monosubstituted or polysubstituted by halogen, -($C_1$-$C_4$)alkyl, ($C_1$-$C_4$)alkoxy, $NO_2$, $CF_3$, CN or $OCHF_2$; or in the event that $R^2$ is ($C_2$-$C_8$)alkenyl, ($C_2$-$C_8$)alkynyl, phenyl, phenoxy, each of which can be substituted as indicated below, ($C_1$-$C_4$)alkylsulfonyl or ($C_1$-$C_4$)alkylsulfinyl and $n \neq 0$, $R^1$ is also ($C_1$-$C_8$)alkyl,

$R^2$ radicals independently of one another are ($C_1$-$C_8$)alkyl, ($C_2$-$C_8$)alkenyl, ($C_2$-$C_8$)-alkynyl, phenyl, phenoxy, ($C_1$-$C_4$)alkoxy, ($C_1$-$C_4$)alkylthio, ($C_1$-$C_4$)alkoxycarbonyl, ($C_2$-$C_4$)alkenyloxycarbonyl or ($C_2$-$C_4$)alkynyloxycarbonyl, it being possible for all of the abovementioned radicals to be monosubstituted or polysubstituted by halogen or monosubstituted to disubstituted by ($C_1$-$C_4$)alkoxy or ($C_1$-$C_4$)alkylthio; furthermore are halogen, $NO_2$, ($C_1$-$C_4$)alkylsulfonyl or ($C_1$-$C_4$)alkylsulfinyl;

n is 0, 1, 2 or 3;

Y is O or S;

$R^3$ is hydrogen, ($C_1$-$C_8$)alkyl, ($C_2$-$C_8$)alkenyl, ($C_3$-$C_8$)alkynyl or ($C_1$-$C_4$)alkoxy;

$R^4$ is a heterocyclic radical of the formulae

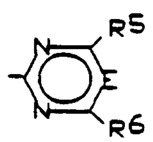 , 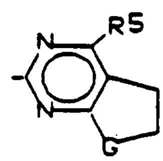 , 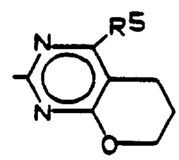 ,

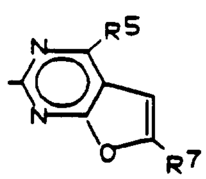 , 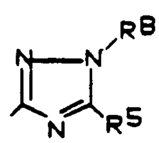 , 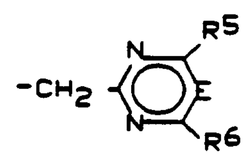 ,

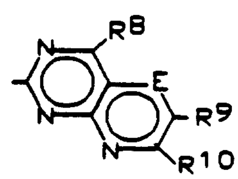 or 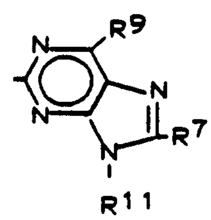

E is CH or N,

G is O or $CH_2$,

$R^5$ and $R^6$ independently of one another are hydrogen, halogen, ($C_1$-$C_6$)alkyl, ($C_1$-$C_6$)alkoxy or ($C_1$-$C_6$)-alkylthio, it being possible for the abovementioned alkyl-containing radicaAls to be monosubstituted or polysubstituted by halogen or monosubstituted or disubstituted by ($C_1$-$C_4$)alkoxy or ($C_1$-$C_4$)alkylthio, furthermore are a radical of the formula $-NR^{12}R^{13}$, $-OCHR^7-CO_2R^{12}$, ($C_3$-$C_6$)cycloalkyl, ($C_3$-$C_5$)alkenyl, ($C_2$-$C_4$)alkynyl, ($C_3$-$C_5$)alkenyloxy or ($C_3$-$C_5$)alkynyloxy,

$R^7$ is hydrogen or ($C_1$-$C_4$)alkyl,

$R^8$ is ($C_1$-$C_4$)alkyl, $-CHF_2$ or $-CH_2CF_3$,

$R^9$ and $R^{10}$ independently of one another are hydrogen, ($C_1$-$C_4$)alkyl, ($C_1$-$C_5$)alkoxy or halogen,

$R^{11}$ is hydrogen, ($C_1$-$C_4$)alkyl, $-CHF_2$ or $CH_2CF_3$ and

$R^{12}$ and $R^{13}$ independently of one another are hydrogen, ($C_1$-$C_4$)alkyl, ($C_2$-$C_4$)alkenyl or ($C_3$-$C_4$)-alkynyl.

**2.** The method as claimed in claim 1, wherein, in formula I, $R^1$ is $(C_1\text{-}C_4)$alkyl, $(C_2\text{-}C_5)$alkenyl or $(C_2\text{-}C_4)$-alkynyl, these radicals being substituted as indicated in claim 1; furthermore $(C_2\text{-}C_5)$alkenyl or $(C_2\text{-}C_4)$-alkynyl or, in the event that n = 1 and $R^2$ is $(C_2\text{-}C_8)$alkenyl or $(C_2\text{-}C_8)$alkynyl, is a $(C_1\text{-}C_4)$alkyl radical; $R^2$ is $(C_1\text{-}C_4)$alkyl, $(C_2\text{-}C_5)$alkenyl, $(C_2\text{-}C_4)$alkynyl, $(C_1\text{-}C_4)$alkoxycarbonyl, phenyl, phenoxy, $(C_1\text{-}C_4)$-alkoxy, $(C_1\text{-}C_4)$alkylthio, it being possible for these radicals to be substituted as indicated in claim 1; or is halogen; n is 0, 1 or 2, Y is O, $R^3$ is hydrogen, $(C_1\text{-}C_4)$alkyl or $(C_3\text{-}C_4)$alkenyl, $R^4$ is a heterocyclic radical of the formula

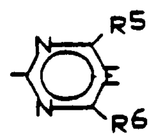 ,

E is CH or N,
$R^5$ and $R^6$ independently of one another are halogen, $(C_1\text{-}C_4)$alkyl, $(C_1\text{-}C_4)$alkoxy or $(C_1\text{-}C_4)$alkylthio, each of which can be substituted as indicated in claim 1.

**3.** The method as claimed in claim 1 or 2, wherein, in formula I, $R^1$ is $(C_1\text{-}C_4)$alkyl, $(C_2\text{-}C_5)$alkenyl, $(C_2\text{-}C_4)$alkynyl, these radicals being substituted as indicated in claim 1, furthermore $(C_2\text{-}C_5)$alkenyl or $(C_2\text{-}C_4)$alkynyl, $R^2$ is $(C_1\text{-}C_4)$alkyl, $(C_2\text{-}C_5)$alkenyl, $(C_1\text{-}C_4)$alkoxycarbonyl, $(C_1\text{-}C_4)$alkoxy or $(C_1\text{-}C_4)$alkylthio, each of which can be substituted as indicated in claim 1; or is fluorine or chlorine, n = 0 or 1, $R^3$ is hydrogen or methyl, $R^4$ is a heterocyclic radical of the formula

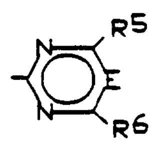

E = CH or N
and $R^5$ and $R^6$ independently of one another are chlorine, bromine, $(C_1\text{-}C_4)$alkyl, $(C_1\text{-}C_4)$alkoxy, $OCHF_2$, $OCH_2CF_3$ or $CF_3$.

**4.** A process for the preparation of a compound of the formula I defined in claim 1, 2 or 3, or a salt thereof, which comprises reacting
(a) a compound of the formula (II)

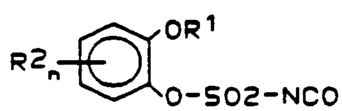 (II)

with a compound of the formula (III)

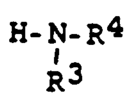 (III),

or

(b) a compound of the formula (IV)

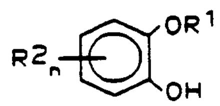

(IV)

with a chlorosulfonylurea of the formula (V)

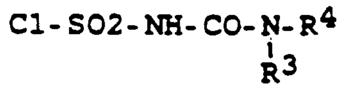

(V),

or

(c) a compound of the formula (VI)

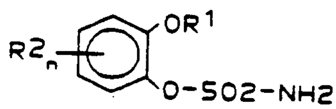

(VI)

with a carbamate of the formula (VII)

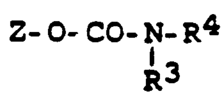

(VII)

where Z is phenyl or $(C_1$-$C_6)$alkyl, and, if desired, converting the resultant compound of the formula I into a salt thereof.

**5.** The method as claimed in claim 1, 2 or 3, wherein between 0.005 and 10 kg/ha of compound of the formula I are applied.

**6.** The use of a compound of the formula I defined in claim 1, 2 or 3, or a salt thereof, as herbicide or plant growth regulator.

**7.** The method as claimed in claim 5, wherein between 0.01 and 5 kg/ha are applied.

**8.** A method for regulating the growth of crop plants, which comprises applying an effective amount of one or more compounds of the formula I defined in claim 1, 2 or 3, or a salt thereof, to these crop plants or the cropped area.

**Revendications**

**Revendications pour les Etats contractants suivants : BE, CH, DE, FR, GB, IT, LI, NL, SE**

**1.** Composés de formule I ou leurs sels

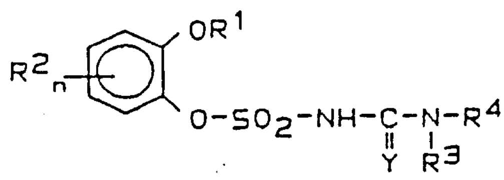

(I),

dans laquelle

$R^1$ est un radical alkyle en $C_1$-$C_8$, alcényle en $C_2$-$C_8$, alcynyle en $C_2$-$C_8$, ces radicaux étant chacun substitués une ou plusieurs fois par le chlore ou le brome, une ou deux fois par des substituants alcoxy en $C_1$-$C_6$, alcényloxy en $C_2$-$C_6$, alcynyloxy en $C_2$-$C_6$, alkylthio en $C_1$-$C_6$,alkylsulfinyle en $C_1$-$C_6$, alkylsulfonyle en $C_1$-$C_6$, (alcoxy en $C_1$-$C_6$)carbonyle, $NO_2$, CN ou par des radicaux phényle ; de plus alcényle en $C_2$-$C_8$, alcynyle en $C_2$-$C_8$, cycloalkyle en $C_3$-$C_8$ pouvant être une ou plusieurs fois substitués par des halogènes ou une ou deux fois substitués par des substituants alcoxy en $C_1$-$C_4$ ou alkylthio en $C_1$-$C_4$ ; cycloalcényle en $C_5$-$C_8$, cyclopropylméthyle, époxypropyle ; furfuryle, tétrahydrofurfuryle, phénoxy(alkyle en $C_1$-$C_4$), ces trois derniers substituants pouvant être substitués par des halogènes ou des radicaux alkyle en $C_1$-$C_4$ ou alcoxy en $C_1$-$C_4$ ; ou phényle, lequel peut être une ou plusieurs fois substitué par des halogènes ou des radicaux alkyle en $C_1$-$C_4$, alcoxy en $C_1$-$C_4$, $NO_2$, $CF_3$, CN ou $OCHF_2$ ; ou, quand $R^2$ est un radical alcényle en $C_2$-$C_8$, alcynyle en $C_2$-$C_8$, phényle, phénoxy, qui peuvent être substitués comme indiqué ci-après, un radical alkylsulfonyle en $C_1$-$C_4$ ou alkylsulfinyle en $C_1$-$C_4$, et n est différent de 0, $R^1$ peut également être un radical alkyle en $C_1$-$C_8$,

les radicaux $R^2$, indépendamment les uns des autres, représentent, chacun, un radical alkyle en $C_1$-$C_8$, alcényle en $C_2$-$C_8$, alcynyle en $C_2$-$C_8$, phényle, phénoxy, alcoxy en $C_1$-$C_4$, alkylthio en $C_1$-$C_4$, (alcoxy en $C_1$-$C_4$)carbonyle, (alcényloxy en $C_2$-$C_4$)carbonyle, (alcynyloxy en $C_2$-$C_4$)carbonyle, tous les radicaux ci-dessus pouvant être une ou plusieurs fois substitués par des halogènes ou une ou deux fois par des radicaux alcoxy en $C_1$-$C_4$ ou alkylthio en $C_1$-$C_4$ ; de plus un halogène, un groupe $NO_2$, alkylsulfonyle en $C_1$-$C_4$ ou alkylsulfinyle en $C_1$-$C_4$ ;

n vaut 0, 1, 2 ou 3 ;

y est O ou S ;

$R^3$ est un hydrogène ou un radical alkyle en $C_1$-$C_8$,alcényle en $C_2$-$C_8$, alcynyle en $C_3$-$C_8$ ou alcoxy en $C_1$-$C_4$ ;

$R^4$ est un radical hétérocyclique ayant les formules suivantes :

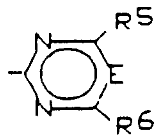 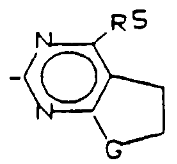, 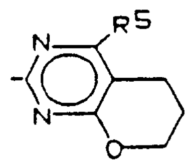,

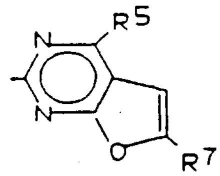, 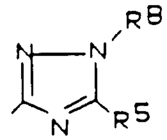, 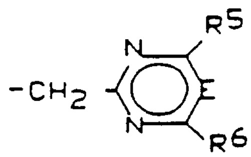,

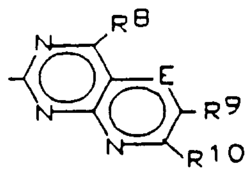 ou 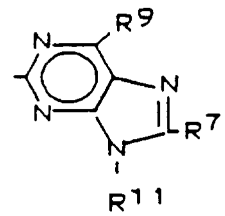

E est CH ou N,
G est O ou $CH_2$,
$R^5$, $R^6$, indépendamment l'un de l'autre, sont chacun un hydrogène, un alogène, un groupe alkyle en $C_1$-$C_6$, alcoxy en $C_1$-$C_6$ du alkylthio en $C_1$-$C_6$, les radicaux alkylés ci-dessus pouvant être une ou plusieurs fois substitués par des halogènes ou une ou deux fois substitués par des radicaux alcoxy en $C_1$-$C_4$ ou alkylthio en $C_1$-$C_4$, de plus un radical de formule $-NR^{12}R^{13}$, $-OCHR^7-CO_2R^{12}$, cycloalkyle en $C_3$-$C_6$, alcényle en $C_3$-$C_5$, alcynyle en $C_2$-$C_4$, alcényloxy en $C_3$-$C_5$ ou alcynyloxy en $C_3$-$C_5$,
$R^7$ est un atome d'hydrogène ou un radical alkyle en $C_1$-$C_4$,
$R^8$ est un radical alkyle en $C_1$-$C_4$, $-CHF_2$ ou $-CH_2CF_3$,
$R^9$, $R^{10}$, indépendamment l'un de l'autre, sont chacun un hydrogène ou un radical alkyle en $C_1$-$C_4$, alcoxy en $C_1$-$C_5$ ou un halogène,
$R^{11}$ est un atome d'hydrogène ou un radical alkyle en $C_1$-$C_4$, $-CHF_2$ ou $CH_2CF_3$, et
$R^{12}$, $R^{13}$, indépendamment l'un de l'autre, sont chacun un atome d'hydrogène ou un radical alkyle en $C_1$-$C_4$,alcényle en $C_2$-$C_4$ ou alcynyle en $C_3$-$C_4$.

**2.** Composés de formule I selon la revendication 1 ou leurs sels, dans lesquels $R^1$ est un radical alkyle en $C_1$-$C_4$,alcényle en $C_2$-$C_5$ ou alcynyle en $C_2$-$C_4$, ces radicaux étant substitués comme indiqué dans la revendication 1 ; en outre un radical alcényle en $C_2$-$C_5$ ou alcynyle en $C_2$-$C_4$ ou, quand n = 1 et $R^2$ est un radical alcényle en $C_2$-$C_8$ ou alcynyle en $C_2$-$C_8$, un radical alkyle $C_1$-$C_4$ ; $R^2$ est un radical alkyle en $C_1$-$C_4$, alcényle en $C_2$-$C_5$, alcynyle en $C_2$-$C_4$, (alcoxy en $C_1$-$C_4$)carbonyle, phényle, phénoxy, alcoxy en $C_1$-$C_4$, alkylthio en $C_1$-$C_4$, ces radicaux pouvant être substitués comme indiqué dans la revendication 1 ; ou un halogène ; n vaut 0, 1 ou 2, Y = O, $R^3$ est un atome d'hydrogène ou un radical alkyle en $C_1$-$C_4$ ou alcényle en $C_3$-$C_4$, $R^4$ est un radical hétérocyclique de formule

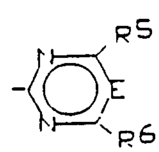,

E = CH ou N,
$R^5$ et $R^6$, indépendamment l'un de l'autre, sont chacun un halogène ou un radical alkyle en $C_1$-$C_4$, alcoxy en $C_1$-$C_4$, alkylthio en $C_1$-$C_4$, qui peuvent être substitués comme indiqué dans la revendication 1.

**3.** Composés de formule I selon la revendication 1 ou leurs sels, dans lesquels $R^1$ est un radical alkyle en $C_1$-$C_4$,alcényle en $C_2$-$C_5$, alcynyle en $C_2$-$C_4$, ces radicaux étant substitués comme indiqué dans la revendication 1, de plus, alcényle en $C_2$-$C_5$ ou alcynyle en $C_2$-$C_4$, $R^2$ est un radical alkyle en $C_1$-$C_4$, alcényle en $C_2$-$C_5$, (alcoxy en $C_1$-$C_4$)carbonyle, alcoxy en $C_1$-$C_4$, alkylthio en $C_1$-$C_4$, qui peuvent être substitués comme indiqué dans la revendication 1 ; un atome de fluor ou de chlore , n vaut 0 ou 1, $R^3$ est un atome d'hydrogène ou le radical méthyle, $R^4$ est un radical hétérocyclique de formule

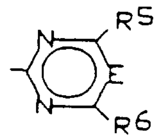

E est CH ou N

et $R^5$ et $R^6$, indépendamment l'un de l'autre, sont chacun un atome de chlore ou de brome, ou un radical alkyle en $C_1$-$C_4$ alcoxy en $C_1$-$C_4$, $OCHF_2$, $OCH_2CF_3$ ou $CF_3$.

**4.** Procédé pour préparer les composés de formule I selon l'une quelconque des revendications 1 à 3 ou leurs sels, caractérisé en ce que

(a) on fait réagir un composé de formule (II)

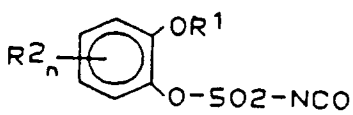

(II)

avec un composé de formule (III)

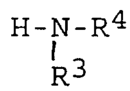

ou

(b) on fait réagir un composé de formule (IV)

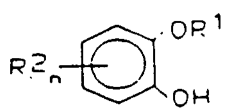

avec une chlorosulfonylurée de formule (V)

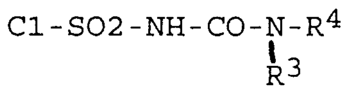

(V)

ou

(c) on fait réagir un composé de formule (VI)

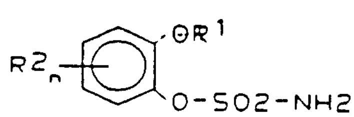

(VI)

avec un carbamate de formule (VII)

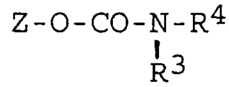

où Z est le radical phényle ou un radical alkyle en $C_1$-$C_6$,les composés de formule I ainsi obtenus étant éventuellement convertis dans leurs sels.

**5.** Herbicides ou agents régulateurs de croissance des végétaux, caractérisés en ce qu'ils contiennent une quantité efficace d'un ou plusieurs composés de formule I, ou leurs sels, selon l'une quelconque des revendications 1 à 3, et des adjuvants inertes.

**6.** Utilisation des composés de formule I ou de leurs sels selon les revendications 1 à 3 en tant qu'herbicides ou régulateurs de croissance des végétaux.

**7.** Procédé pour maîtriser les mauvaises herbes, caractérisé en ce qu'on applique sur ces dernières, ou sur des sols à usage agricole ou industriel, une quantité efficace d'un ou plusieurs composés de formule I ou de leurs sels selon l'une quelconque des revendications 1 à 3.

**8.** Procédé pour réguler la croissance de plantes de culture, caractérisé en ce qu'on applique sur ces dernières ou sur des surfaces de culture une quantité efficace d'un ou plusieurs composés de formule I ou de leurs sels selon l'une des revendications 1 à 3.

**Revendications pour l'Etat contractant suivant : ES**

**1.** Procédé pour maîtriser les mauvaises herbes, caractérisé en ce qu'on applique sur ces dernières, ou sur les sols à usage agricole ou industriel, une quantité efficace d'un ou plusieurs composés de formule I ou leurs sels

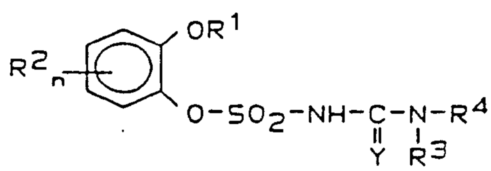 (I),

dans laquelle

$R^1$ est un radical alkyle en $C_1$-$C_8$, alcényle en $C_2$-$C_8$, alcynyle en $C_2$-$C_8$, ces radicaux étant chacun substitués une ou plusieurs fois par le chlore ou le brome, une ou deux fois par des substituants alcoxy en $C_1$-$C_6$, alcényloxy en $C_2$-$C_6$, alcynyloxy en $C_2$-$C_6$, alkylthio en $C_1$-$C_6$,alkylsulfinyle en $C_1$-$C_6$, alkylsulfonyle en $C_1$-$C_6$, (alcoxy en $C_1$-$C_6$)carbonyle, $NO_2$, CN ou par des radicaux phényle ; de plus alcényle en $C_2$-$C_8$, alcynyle en $C_2$-$C_8$, cycloalkyle en $C_3$-$C_8$ pouvant être une ou plusieurs fois substitués par des halogènes ou une ou deux fois substitués par des substituants alcoxy en $C_1$-$C_4$ ou alkylthio en $C_1$-$C_4$ ; cycloalcényle en $C_5$-$C_8$, cyclopropylméthyle, époxypropyle ; furfuryle, tétrahydrofurfuryle, phénoxy(alkyle en $C_1$-$C_4$), ces trois derniers substituants pouvant être substitués par des halogènes ou des radicaux alkyle en $C_1$-$C_4$ ou alcoxy en $C_1$-$C_4$ ; ou phényle, lequel peut être une ou plusieurs fois substitué par des halogènes ou des radicaux alkyle en $C_1$-$C_4$, alcoxy en $C_1$-$C_4$, $NO_2$, $CF_3$, CN ou $OCHF_2$ ; ou, quand $R^2$ est un radical alcényle en $C_2$-$C_8$, alcynyle en $C_2$-$C_8$, phényle, phénoxy, qui peuvent être substitués comme indiqué ci-après, un radical alkylsulfonyle en $C_1$-$C_4$ ou alkylsulfinyle en $C_1$-$C_4$, et, n est différent de 0, $R^1$ peut également être un radical alkyle en $C_1$-$C_8$,

les radicaux $R^2$, indépendamment les uns des autres, représentent, chacun, un radical alkyle en $C_1$-$C_8$, alcényle en $C_2$-$C_8$, alcynyle en $C_2$-$C_8$, phényle, phénoxy, alcoxy en $C_1$-$C_4$, alkylthio en $C_1$-$C_4$, (alcoxy en $C_1$-$C_4$)carbonyle, (alcényloxy en $C_2$-$C_4$)carbonyle, (alcynyloxy en $C_2$-$C_4$) oxycarbonyle, tous les radicaux ci-dessus pouvant être une ou plusieurs fois substitués par des halogènes ou une ou deux fois par des radicaux alcoxy en $C_1$-$C_4$ ou alkylthio en $C_1$-$C_4$ ; de plus un atome d'alogène, ou un

radical $NO_2$, alkylsulfonyle en $C_1$-$C_4$ ou alkylsulfinyle en $C_1$-$C_4$ ;

n vaut 0, 1, 2 ou 3 ;

y est O ou S ;

$R^3$ est un hydrogène ou un radical alkyle en $C_1$-$C_8$,alcényle en $C_2$-$C_8$, alcynyle en $C_3$-$C_8$ ou alcoxy en $C_1$-$C_4$ ;

$R^4$ est un radical hétérocyclique ayant les formules suivantes :

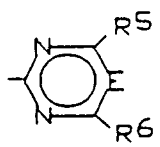 , 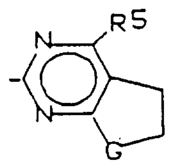 , 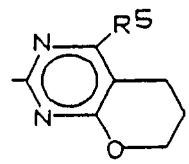 ,

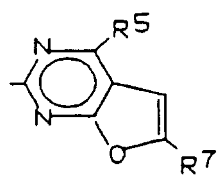 , 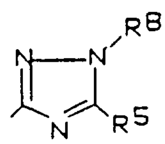 , 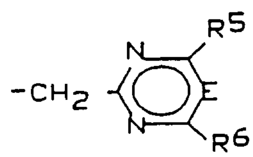 ,

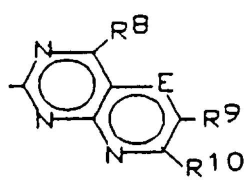 ou 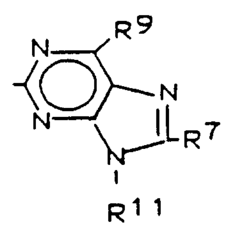

E est CH ou N,

G est O ou $CH_2$,

$R^5$ , $R^6$, indépendamment l'un de l'autre, sont chacun un hydrogène, un halogène, un groupe alkyle en $C_1$-$C_6$, alcoxy en $C_1$-$C_6$ ou alkylthio en $C_1$-$C_6$, les radicaux alkylés ci-dessus pouvant être une ou plusieurs fois substitués par des halogènes ou une ou deux fois substitués par des radicaux alcoxy en $C_1$-$C_4$ ou alkylthio en $C_1$-$C_4$, de plus un radical de formule $-NR^{12}R^{13}$, $-OCHR^7-CO_2R^{12}$, cycloalkyle en $C_3$-$C_6$, alcényle en $C_3$-$C_5$, alcynyle en $C_2$-$C_4$, alcényloxy en $C_3$-$C_5$ ou alcynyloxy en $C_3$-$C_5$,

$R^7$ est un atome d'hydrogène ou un radical alkyle en $C_1$-$C_4$,

$R^8$ est un radical alkyle en $C_1$-$C_4$, $-CHF_2$ ou $-CH_2CF_3$,

$R^9$ , $R^{10}$, indépendamment l'un de l'autre, sont chacun un hydrogène ou un radical alkyle en $C_1$-$C_4$, alcoxy en $C_1$-$C_5$ ou un halogène,

$R^{11}$ est un atome d'hydrogène ou un radical alkyle en $C_1$-$C_4$, $-CHF_2$ ou $CH_2CF_3$, et

$R^{12}$ , $R^{13}$, indépendamment l'un de l'autre, sont chacun un atome d'hydrogène ou un radical alkyle en $C_1$-$C_4$,alcényle en $C_2$-$C_4$ ou alcynyle en $C_3$-$C_4$.

**2.** Procédé selon la revendication 1, caractérisé en ce que, dans la formule I, $R^1$ est un radical alkyle en $C_1$-$C_4$,alcényle en $C_2$-$C_5$ ou alcynyle en $C_2$-$C_4$, ces radicaux étant substitués comme indiqué dans la revendication 1 ; en outre un radical alcényle en $C_2$-$C_5$ ou alcynyle en $C_2$-$C_4$ ou, quand n = 1 et $R^2$ est un radical alcényle en $C_2$-$C_8$ ou alcynyle en $C_2$-$C_8$, un radical alkyle $C_1$-$C_4$ ; $R^2$ est un radical alkyle en $C_1$-$C_4$, alcényle en $C_2$-$C_5$, alcynyle en $C_2$-$C_4$, (alcoxy en $C_1$-$C_4$)carbonyle, phényle, phénoxy, alcoxy en $C_1$-$C_4$, alkylthio en $C_1$-$C_4$, ces radicaux pouvant être substitués comme indiqué dans la revendication 1 ; ou un halogène ; n vaut 0, 1 ou 2, Y = O, $R^3$ est un atome d'hydrogène ou un radical alkyle en $C_1$-$C_4$ ou alcényle en $C_3$-$C_4$, $R^4$ est un radical hétérocyclique de formule

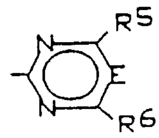

E = CH ou N,

$R^5$ et $R^6$, indépendamment l'un de l'autre, sont chacun un halogène ou un radical alkyle en $C_1$-$C_4$, alcoxy en $C_1$-$C_4$, alkylthio en $C_1$-$C_4$, qui peuvent être substitués comme indiqué dans la revendication 1.

**3.** Procédé selon la revendication 1 ou 2, caractérisé en ce que, dans la formule I, $R^1$ est un radical alkyle en $C_1$-$C_4$, alcényle en $C_2$-$C_5$, alcynyle en $C_2$-$C_4$, ces radicaux étant substitués comme indiqué dans la revendication 1, de plus, alcényle en $C_2$-$C_5$ ou alcynyle en $C_2$-$C_4$, $R^2$ est un radical alkyle en $C_1$-$C_4$, alcényle en $C_2$-$C_5$, (alcoxy en $C_1$-$C_4$)carbonyle, alcoxy en $C_1$-$C_4$, alkylthio en $C_1$-$C_4$, qui peuvent être substitués comme indiqué dans la revendication 1 ; un atome de fluor ou de chlore ; n vaut 0 ou 1, $R^3$ est un atome d'hydrogène ou le radical méthyle, $R^4$ est un radical hétérocyclique de formule

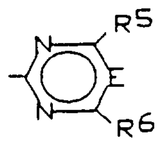

E est CH ou N

et $R^5$ et $R^6$, indépendamment l'un de l'autre, sont chacun un atome de chlore ou de brome, ou un radical alkyle en $C_1$-$C_4$, alcoxy en $C_1$-$C_4$, $OCHF_2$, $OCHF_2CF_3$ ou $CF_3$.

**4.** Procédé pour préparer les composés ayant la formule I définie dans les revendications 1, 2 ou 3, ou leurs sels, caractérisé en ce que

(a) on fait réagir un composé de formule (II)

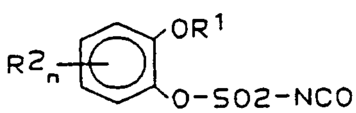

(II)

avec un composé de formule (III)

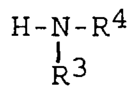

ou

(b) on fait réagir un composé de formule (IV)

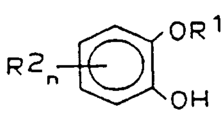

(IV)

avec une chlorosulfonylurée de formule (V)

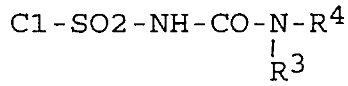

(V)

ou

(c) on fait réagir un composé de formule (VI)

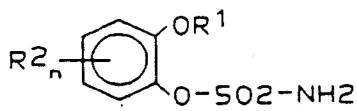

(VI)

avec un carbamate de formule (VII)

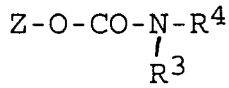

où Z est le radical phényle ou un radical alkyle en $C_1$-$C_6$,les composés de formule I ainsi obtenus étant éventuellement convertis dans leurs sels.

**5.** Procédé selon la revendications 1, 2 ou 3, caractérisé en ce qu'on applique entre 0,005 et 10 kg/ha du composé de formule I.

**6.** Utilisation des composés de formule I définie dans la revendication 1, 2 ou 3 ou de leurs sels en tant qu'herbicides ou régulateurs de croissance des végétaux.

**7.** Procédé selon la revendication 5, caractérisé en ce qu'on applique de entre 0,01 et 5 kg/ha.

**8.** Procédé pour réguler la croissance des plantes de culture, caractérisé en ce qu'on applique sur ces dernières ou sur la surface cultivée une quantité efficace d'un ou plusieurs composés ayant la formule I définie dans la revendication 1, 2 ou 3, ou de leurs sels.